# EUROPEAN PATENT SPECIFICATION

(11) **EP 2 877 190 B1**
(45) Date of publication and mention of the grant of the patent: **02.11.2016**
(21) Application number: 13745543.2
(22) Date of filing: 26.07.2013
(51) Int. Cl.: A61K 35/747, A61K 38/44, A61K 48/00, A61P 3/00

(54) **GENETICALLY MODIFIED PROBIOTIC FOR THE TREATMENT OF PHENYLKETONURIA (PKU) DISEASE**
GENETISCH MODIFIZIERTES PROBIOTIKUM ZUR BEHANDLUNG DER KRANKHEIT PHENYLKETONURIE (PKU)
PROBIOTIQUE GÉNÉTIQUEMENT MODIFIÉ (GMP) POUR LE TRAITEMENT DE LA PHÉNYLCÉTONURIE (PKU)

(30) Priority: 27.07.2012 US 201261676461 P
(43) Date of publication of application: 03.06.2015
(73) Proprietor: University of North Texas, Denton, Texas 76207 (US)
(72) Inventor: DEMING, Katherine, Irving, TX 75061 (US); ALLEN, Michael, Denton, TX 76209 (US); VON HERBING, Ione, Hunt, Denton, TX 76210 (US)
(74) Representative: Tomkins & Co
(86) International application number: PCT/US2013/052200
(87) International publication number: WO 2014/018832

(56) References cited:
- US-A1- 2008 008 695
- US-A1- 2009 047 265
- JIA X ET AL: "[A new strategy of gene therapy for hyperphenylalaninemia rats]", ZHONGHUA YIXUE ZAZHI - NATIONAL MEDICAL JOURNAL OF CHINA, BEIJING, CH, vol. 80, no. 6, 1 June 2000 (2000-06-01), pages 464-467, XP009172888, ISSN: 0376-2491
- LIU JINGZHONG ET AL: "Study on a novel strategy to treatment of phenylketonuria", ARTIFICIAL CELLS, BLOOD SUBSTITUTES, AND IMMOBILIZATION BIOTECHNOLOGY, MARCEL DEKKER INC, US, vol. 30, no. 4, 1 January 2002 (2002-01-01), pages 243-257, XP009172887, ISSN: 1073-1199
- JIA XING-YUAN ET AL: "Food grade expression of artificial phenylalanine-amnonia-lyase gene in Lactococcus lactis", ZHONGHUA WEISHENGWUXUE HE MIANYIXUE ZAZHI - CHINESE JOURNAL OF MICROBIOLOGY AND IMMUNOLOGY, WEISHENG BU YAOPIN SHENGWU ZHIPIN JIANDINGSUO, BEIJING, CN, vol. 26, no. 1, 30 January 2006 (2006-01-30), pages 23-26, XP009172954, ISSN: 0254-5101
- CHEN X ET AL: "High-level Expression of Phenylalanine Ammonia-lyase in Lactococcus lactis via Synthesized Sequence Based on Bias Codons", CHINESE JOURNAL OF BIOTECHNOLOGY,, vol. 22, no. 2, 1 March 2006 (2006-03-01), pages 187-190, XP022857531, ISSN: 1872-2075, DOI: 10.1016/S1872-2075(06)60022-8 [retrieved on 2006-03-01]

### SEQUENCE LISTING

<110> Deming, Katherine
<120> GENETICALLY MODIFIED PROBIOTIC
<130> UNTD-0075 PRO
<160> 15
<170> PatentIn version 3.5
<210> 1
   <211> 14
   <212> DNA
   <213> Artificial Sequence
<220>
   <223> Sequence to be added to to front of codon optimized AvPAL sequence.
<400> 1
   aggagtaggt ataa 14
<210> 2
   <211> 56
   <212> DNA
   <213> Artificial Sequence
<220>
   <223> Sequence to be added to the end of AvPAL sequence.
<400> 2
   tgataagtta aggggtgcat aaactgcatc ccttaactta tcaaaaaaaa gtcgac 56
<210> 3
   <211> 1777
   <212> DNA
   <213> Artificial Sequence
<220>
   <223> Synthesized AvPAL 5' to 3'
<400> 3
   aggagtaggt ataaatgaag actttatctc aagcacaatc taagactagt tctcaacaat      60
   tctcatttac tggaaactca agtgctaatg ttatcattgg taatcaaaag cttactatta     120
   acgatgttgc acgggttgca cgcaatggta ctttagttag tttaactaac aacactgaca     180
   ttttacaagg cattcaagct agttgcgact atatcaataa cgctgttgaa tcaggagaac     240
   caatttatgg tgttacttca ggattcggtg gaatggcaaa cgtcgctatt agtcgtgaac     300
   aagcttctga acttcaaact aatcttgttt ggtttcttaa aacaggagct ggtaataaac     360
   ttccattagc agatgttcgt gctgctatgt tattacgtgc aaattcacac atgcgtggcg     420
   cttctggtat tcgtcttgaa ttaattaagc gtatggaaat tttccttaac gccggtgtta     480
   caccttatgt ttacgaattc ggatcaattg gtgcaagtgg agatcttgtt cctttatctt     540
   atattacagg ttctttaatt ggtttggatc catcattcaa agttgatttt aatggtaaag 600
   aaatggatgc cccaacagct cttcgacaat taaatttatc accacttaca ttacttccaa 660
   aagaaggtct tgctatgatg aatggtacat cagttatgac gggaattgct gctaattgcg 720
   tttatgatac tcaaattctt actgctattg ccatgggtgt tcatgcctta gatattcaag 780
   cacttaatgg cacaaatcaa agtttccacc cattcattca caattctaaa cctcacccag 840
   gtcaactttg ggctgctgat caaatgattt ctcttttagc taatagtcaa cttgttcgcg 900
   acgaattaga tggtaagcat gattatcgtg atcatgaact tattcaagac cgctatagtt 960
   tacgttgctt accacaatat ttgggtccta ttgttgatgg aatttcacaa attgctaagc 1020
   aaattgaaat cgaaattaat agtgtcactg acaatccatt aattgacgtt gataaccaag 1080
   cctcatacca tggcggtaac tttttgggtc aatatgttgg catgggcatg gaccacttac 1140
   gttactacat tggtcttctt gcaaagcatt tggatgtcca aattgcactt ttggccagtc 1200
   cagaattcag taacggactt ccaccatcat tattgggaaa ccgggaacga aaggtaaaca 1260
   tgggtcttaa gggtttgcaa atttgtggca actcaattat gccattgttg actttttacg 1320
   gaaattcaat tgctgatcgc tttcctacac acgccgaaca atttaaccaa aatattaact 1380
   ctcaaggata cacttctgct acgttagcac gacggtctgt tgatattttt caaaactacg 1440
   tagctattgc attaatgttt ggagtacaag ctgttgattt acggacttac aaaaagacgg 1500
   gccattatga tgctcgtgct tgtttaagtc cagctacaga acgtctttat tcagcagtac 1560
   gtcatgttgt cggccaaaaa cctacatcag atcggccata tatttggaac gataacgaac 1620
   aaggacttga cgaacatatt gctcgtattt ctgctgatat cgctgctggt ggtgtaattg 1680
   ttcaagccgt acaagatatc cttccttgtt tacactaata atgataagtt aaggggtgca 1740
   taaactgcat cccttaactt atcaaaaaaa agtcgac 1777
<210> 4
   <211> 186
   <212> DNA
   <213> Artificial Sequence
<220>
   <223> Synthetic ErmB promoter sequence
<400> 4
   gcatgctagc tatagttcta gaggatacct ggttgattaa cgttagcctg gctacgtata 60
   ctcctggaag tattaataga cgacctagga tgcatatgtt caagagtgtg ttgatagtgc 120
   agtatcttaa aattttgtat aataggaatt gaagttaaat tagatgctaa aaatttgtaa 180
   ttaaga 186
<210> 5
   <211> 129
   <212> DNA
   <213> Artificial Sequence
<220>
   <223> Secretion tag sequence
<400> 5
   atgtatactg aaaatacagg taaacatcac cgtaacggac ttcctgtgtg gctattacca       60
   ttattggtcg ttattagttt ttggggcgta agccaaaata ttatggttgt tgatgcttct      120
   tcagttgat                                                              129
<210> 6
   <211> 2092
   <212> DNA
   <213> Artificial Sequence
<220>
   <223> Full secFuzErmAvPAL sequence
<400> 6
   gcatgctagc tatagttcta gaggatacct ggttgattaa cgttagcctg gctacgtata 60
   ctcctggaag tattaataga cgacctagga tgcatatgtt caagagtgtg ttgatagtgc 120
   agtatcttaa aattttgtat aataggaatt gaagttaaat tagatgctaa aaatttgtaa 180
   ttaagaagga gtaggtataa atgtatactg aaaatacagg taaacatcac cgtaacggac 240
   ttcctgtgtg gctattacca ttattggtcg ttattagttt ttggggcgta agccaaaata 300
   ttatggttgt tgatgcttct tcagttgata tgaagacttt atctcaagca caatctaaga 360
   ctagttctca acaattctca tttactggaa actcaagtgc taatgttatc attggtaatc 420
   aaaagcttac tattaacgat gttgcacggg ttgcacgcaa tggtacttta gttagtttaa 480
   ctaacaacac tgacatttta caaggcattc aagctagttg cgactatatc aataacgctg 540
   ttgaatcagg agaaccaatt tatggtgtta cttcaggatt cggtggaatg gcaaacgtcg 600
   ctattagtcg tgaacaagct tctgaacttc aaactaatct tgtttggttt cttaaaacag 660
   gagctggtaa taaacttcca ttagcagatg ttcgtgctgc tatgttatta cgtgcaaatt 720
   cacacatgcg tggcgcttct ggtattcgtc ttgaattaat taagcgtatg gaaattttcc 780
   ttaacgccgg tgttacacct tatgtttacg aattcggatc aattggtgca agtggagatc 840
   ttgttccttt atcttatatt acaggttctt taattggttt ggatccatca ttcaaagttg 900
   attttaatgg taaagaaatg gatgccccaa cagctcttcg acaattaaat ttatcaccac 960
   ttacattact tccaaaagaa ggtcttgcta tgatgaatgg tacatcagtt atgacgggaa 1020
   ttgctgctaa ttgcgtttat gatactcaaa ttcttactgc tattgccatg ggtgttcatg 1080
   ccttagatat tcaagcactt aatggcacaa atcaaagttt ccacccattc attcacaatt 1140
   ctaaacctca cccaggtcaa ctttgggctg ctgatcaaat gatttctctt ttagctaata 1200
   gtcaacttgt tcgcgacgaa ttagatggta agcatgatta tcgtgatcat gaacttattc     1260
   aagaccgcta tagtttacgt tgcttaccac aatatttggg tcctattgtt gatggaattt     1320
   cacaaattgc taagcaaatt gaaatcgaaa ttaatagtgt cactgacaat ccattaattg     1380
   acgttgataa ccaagcctca taccatggcg gtaacttttt gggtcaatat gttggcatgg     1440
   gcatggacca cttacgttac tacattggtc ttcttgcaaa gcatttggat gtccaaattg     1500
   cacttttggc cagtccagaa ttcagtaacg gacttccacc atcattattg ggaaaccggg     1560
   aacgaaaggt aaacatgggt cttaagggtt tgcaaatttg tggcaactca attatgccat     1620
   tgttgacttt ttacggaaat tcaattgctg atcgctttcc tacacacgcc gaacaattta     1680
   accaaaatat taactctcaa ggatacactt ctgctacgtt agcacgacgg tctgttgata     1740
   tttttcaaaa ctacgtagct attgcattaa tgtttggagt acaagctgtt gatttacgga     1800
   cttacaaaaa gacgggccat tatgatgctc gtgcttgttt aagtccagct acagaacgtc     1860
   tttattcagc agtacgtcat gttgtcggcc aaaaacctac atcagatcgg ccatatattt     1920
   ggaacgataa cgaacaagga cttgacgaac atattgctcg tatttctgct gatatcgctg     1980
   ctggtggtgt aattgttcaa gccgtacaag atatccttcc ttgtttacac taataatgat     2040
   aagttaaggg gtgcataaac tgcatccctt aacttatcaa aaaaaagtcg ac             2092
<210> 7
   <211> 7068
   <212> DNA
   <213> Artificial Sequence
<220>
   <223> pSLERGT sequence
<400> 7
   tcttccgctt cctcgctcac tgactcgctg cgctcggtcg ttcggctgcg gcgagcggta      60
   tcagctcact caaaggcggt aatacggtta tccacagaat caggggataa cgcaggaaag     120
   aacatgtgag caaaaggcca gcaaaaggcc aggaaccgta aaaaggccgc gttgctggcg     180
   tttttccata ggctccgccc ccctgacgag catcacaaaa atcgacgctc aagtcagagg     240
   tggcgaaacc cgacaggact ataaagatac caggcgtttc cccctggaag ctccctcgtg     300
   cgctctcctg ttccgaccct gccgcttacc ggatacctgt ccgcctttct cccttcggga     360
   agcgtggcgc tttctcatag ctcacgctgt aggtatctca gttcggtgta ggtcgttcgc     420
   tccaagctgg gctgtgtgca cgaacccccc gttcagcccg accgctgcgc cttatccggt     480
   aactatcgtc ttgagtccaa cccggtaaga cacgacttat cgccactggc agcagccact     540
   ggtaacagga ttagcagagc gaggtatgta ggcggtgcta cagagttctt gaagtggtgg     600
   cctaactacg gctacactag aagaacagta tttggtatct gcgctctgct gaagccagtt     660
   accttcggaa aaagagttgg tagctcttga tccggcaaac aaaccaccgc tggtagcggt      720
   ggtttttttg tttgcaagca gcagattacg cgcagaaaaa aaggatctca agaagatcct      780
   ttgatctttt ctacggggtc tgacgctcag tggaacgaaa actcacgtta agggattttg      840
   gtcatgagat tatcaaaaag gatcttcacc tagatccttt taaattaaaa atgaagtttt      900
   aaatcaatct aaagtatata tgagtaaact tggtctgaca gttaccaatg cttaatcagt      960
   gaggcaccta tctcagcgat ctgtctattt cgttcatcca tagttgcctg actccccgtc     1020
   gtgtagataa ctacgatacg ggagggctta ccatctggcc ccagtgctgc aatgataccg     1080
   cgagacccac gctcaccggc tccagattta tcagcaataa accagccagc cggaagggcc     1140
   gagcgcagaa gtggtcctgc aactttatcc gcctccatcc agtctattaa ttgttgccgg     1200
   gaagctagag taagtagttc gccagttaat agtttgcgca acgttgttgc cattgctaca     1260
   ggcatcgtgg tgtcacgctc gtcgtttggt atggcttcat tcagctccgg ttcccaacga     1320
   tcaaggcgag ttacatgatc ccccatgttg tgcaaaaaag cggttagctc cttcggtcct     1380
   ccgatcgttg tcagaagtaa gttggccgca gtgttatcac tcatggttat ggcagcactg     1440
   cataattctc ttactgtcat gccatccgta agatgctttt ctgtgactgg tgagtactca     1500
   accaagtcat tctgagaata gtgtatgcgg cgaccgagtt gctcttgccc ggcgtcaata     1560
   cgggataata ccgcgccaca tagcagaact ttaaaagtgc tcatcattgg aaaacgttct     1620
   tcggggcgaa aactctcaag gatcttaccg ctgttgagat ccagttcgat gtaacccact     1680
   cgtgcaccca actgatcttc agcatctttt actttcacca gcgtttctgg gtgagcaaaa     1740
   acaggaaggc aaaatgccgc aaaaaaggga ataagggcga cacggaaatg ttgaatactc     1800
   atactcttcc tttttcaata ttattgaagc atttatcagg gttattgtct catgagcgga     1860
   tacatatttg aatgtattta gaaaaataaa caaatagggg ttccgcgcac atttccccga     1920
   aaagtgccac ctgacgtcta agaaaccatt attatcatga cattaaccta taaaaatagg     1980
   cgtatcacga ggccctttcg tctcgcgcgt ttcggtgatg acggtgaaaa cctctgacac     2040
   atgcagctcc cggagacggt cacagcttgt ctgtaagcgg atgccgggag cagacaagcc     2100
   cgtcagggcg cgtcagcggg tgttggcggg tgtcggggct ggcttaacta tgcggcatca     2160
   gagcagattg tactgagagt gcaccataaa attgtaaacg ttaatatttt gttaaaattc     2220
   gcgttaaatt tttgttaaat cagctcattt tttaaccaat aggccgaaat cggcaaaatc     2280
   ccttataaat caaaagaata gcccgagata gggttgagtg ttgttccagt ttggaacaag     2340
   agtccactat taaagaacgt ggactccaac gtcaaagggc gaaaaaccgt ctatcagggc     2400
   gatggcccac tacgtgaacc atcacccaaa tcaagttttt tggggtcgag gtgccgtaaa     2460
   gcactaaatc ggaaccctaa agggagcccc cgatttagag cttgacgggg aaagccggcg     2520
   aacgtggcga gaaaggaagg gaagaaagcg aaaggagcgg gcgctagggc gctggcaagt     2580
   gtagcggtca cgctgcgcgt aaccaccaca cccgccgcgc ttaatgcgcc gctacagggc 2640
   gcgtactatg gttgctttga cgtatgcggt gtgaaatacc gcacagatgc gtaaggagaa 2700
   aataccgcat caggcgccat tcgccattca ggctgcgcaa ctgttgggaa gggcgatcgg 2760
   tgcgggcctc ttcgctatta cgccagctgg cgaaaggggg atgtgctgca aggcgattaa 2820
   gttgggtaac gccagggttt tcccagtcac gacgttgtaa aacgacggcc agtgaattcg 2880
   aatggccatg gaggatttat tctctccgca gttttgagct taatcgtctc attaactact 2940
   gcacttttct ccgattggct tcgtcggaga aaataatcta acggcatgaa gcgcaaatta 3000
   aaagccactc aactatgggc ctagttgggt ggcttttttg caaccattac ggttttactt 3060
   ctctataaga attatagcat caatcttcat cattagccaa ctcaatagaa gctcgatata 3120
   gatcatgttg agctttatcc aagctatcta aaagttcgcc ataaatatca atatttttct 3180
   tttcagcaac caattttttc aaatcttgaa gattatcagc aatctgttct gccataactc 3240
   catagatttc agcttgtttc ttcatattgt caattgtctt aatatcttgt aactccattc 3300
   ctatcactcc catcttacat agttacttac cttagaattc caacgatata tcacagtatc 3360
   tacaacatca tctttttctt tttcgtcatc cacattaatt agatcatctt ctttttcgtc 3420
   cagcaataac tcatgatgaa tctcttttag taatcccgca aagcttaatt gtcgagttcc 3480
   agccagagca tgctcgagct cgtcaacaac aactaaatcg cctttctcat catccgtaat 3540
   ataatcataa tctttaactt gatatttact tacttctttt gccgaagcaa tcaaactatt 3600
   atcttttttt gacgcccgaa ttttcttgat attaacaatc ggcttgtagt ctaatttcct 3660
   tgctcgcttc cataattgtg accaattctc ttgggtaaga taatgaccct tagaaaaata 3720
   actagatttc accattaaca aaatatgtac atgttgatgg aaagttagac tatctcgatt 3780
   gaccgttatt tctgtagatc gcacataacc aagaagatct ttttttacat ctttatattg 3840
   aaaaagccga taaattgaac gattcatcga agtaagattt tttctaagct cgcccaactt 3900
   tgagctttcc tcagttaatg tcaagaacag aaatatagca ctaggatcct ttttatatgc 3960
   tgtatctaaa atttgacgca actcataaga atttttcatc gatcggcgcc agctacataa 4020
   tggacaaagt ctcgaatgac aaaaccacgt ttgatataac tttaaaccat tatcagtttt 4080
   cgcaaattga agaacttctc cgcactttga aacgttatga gcttttttaa aatttaaaac 4140
   ctccagatat tgggaataag ttaagttagc aagttttttc tgcttccacg gtctaacttt 4200
   cccattacta gaggtatctt ttagaatttc ttttttatac atttcttata cctctaactg 4260
   caaaaactac aagcagtcag agttagttat cattgatata ttcctaatga catattataa 4320
   tagggttcga agttaataac gataactaac tcatgcatcg acggccaaat cgaacaactg 4380
   catgagtttt ttatttaatt cttacaatgc ttattataca ccaaaaagtg ttatatttta 4440
   aaatcctaat aaacccagtc atatcaaggg tttaacccaa tttcaaaaaa tggcgttgtt 4500
   tctatatgta tcaagataag aagaaactcg cttacagcga gtttctaaat aggggtttca 4560
   cccctaaaac cctgaccagc tgagccagct ggaccgcatt tcccctcgcc gggcgggcgt 4620
   aaagcggaaa aaatgacttt tttctcgctt tcgctcaact agggtcttcc cgtctcacca 4680
   tgtcaagcgc tgcccttccg ctacgctcca ggcgtttgtc gcttgacact cacctactaa 4740
   cagcgggctt cgccaccagt ctactatctg caacacggca acaagattaa tcagaatatg 4800
   cattgaagca gtgcgctccg ctccttgtct tgcgacaaag cgagcgcact gcttcaatgc 4860
   ttttttattt tctgaattcc ataactcgat aattagaaaa agttagtccg ttagaaacgt 4920
   ctgccacgcc gaggaagtca ttccagtttg accactcaaa tcatcagcga catcattgag 4980
   ataatcaatc agcaattttc tagtttctat ctgctttgga atttttacca cagccaaaat 5040
   tgagtcccac ttagcaatca caaaagattt tctgacggct ttattagcct tttccgcact 5100
   aatatttttc gctttatctc gtttattttg atcggtcgat ggtatccgat cgactgttgt 5160
   tacagcattt gacgcaggtt gctagataaa cgactagacc gaataatttt tattccaact 5220
   atttttgcaa ccaaaaaaat cacaaaataa tcaaccaata aaacgataac taacttcttg 5280
   ctacatccga ttaatttttc tccagcctgc cagaaatcgg cccaaatatt gaaatattga 5340
   ggaaattttg ggctgaaaaa aacattaatt ttttcggcaa acaagccaac acagccaagc 5400
   agaaaagcta agaaaataag ttccagccaa ttaaaaaatc gccacgaaag agaaaaaaat 5460
   cggtcaaaat aaatcgaact tgatttcatt ttctagcccc cgaagtcttc gataaacggc 5520
   gccataaaag gaactacacg gttttccggt tgagtacttg caagccctaa ccccttagaa 5580
   aagccgtcat gattaacaac tgttgtcgac tcagctaggg ccctatatat ggatccaatt 5640
   gcaatgatca tcatgacaga tctgcgcgcg atcgatatca gcgctttaaa tttgcgcatg 5700
   ctagctatag ttctagaggt accggttgtt aacgttagcc ggctacgtat actccggaat 5760
   attaataggc ctaggatgca tatgcttaga agcaaactta agagtgtgtt gatagtgcag 5820
   tatcttaaaa ttttgtataa taggaattga agttaaatta gatgctaaaa atttgtaatt 5880
   aagaaggagt gattacatga acaaaaatat aaaatattct caaaactttt taacgagtga 5940
   aaaagtactc aaccaaataa taaaacaatt gaatttaaaa gaaaccgata ccgtttacga 6000
   aattggaaca ggtaaagggc atttaacgac gaaactggct aaaataagta aacaggtaac 6060
   gtctattgaa ttagacagtc atctattcaa cttatcgtca gaaaaattaa aactgaatac 6120
   tcgtgtcact ttaattcacc aagatattct acagtttcaa ttccctaaca aacagaggta 6180
   taaaattgtt gggagtattc cttaccattt aagcacacaa attattaaaa aagtggtttt 6240
   tgaaagccat gcgtctgaca tctatctgat tgttgaagaa ggattctaca agcgtacctt 6300
   ggatattcac cgaacactag ggttgctctt gcacactcaa gtctcgattc agcaattgct 6360
   taagctgcca gcggaatgct ttcatcctaa accaaaagta aacagtgtct taataaaact 6420
   tacccgccat accacagatg ttccagataa atattggaag ctatatacgt actttgtttc 6480
   aaaatgggtc aatcgagaat atcgtcaact gtttactaaa aatcagtttc atcaagcaat 6540
   gaaacacgcc aaagtaaaca atttaagtac cgttacttat gagcaagtat tgtctatttt 6600
   taatagttat ctattattta acgggaggaa ataattctat gagtcgcttt tgtaaatttg 6660
   gaaagttaca cgttactaaa gggaatgtag ataaattatt aggtatacta ctgacagctt 6720
   ccaagcatat ggcggccgcc tgcagctggc gccatcgata cgcgtacgtc gcgaccgcgg 6780
   acatgtacag agctcgagaa gtactagtgg ccacgtgggc cgtgcacctt aagcttggcg 6840
   taatcatggt catagctgtt tcctgtgtga aattgttatc cgctcacaat tccacacaac 6900
   atacgagccg gaagcataaa gtgtaaagcc tggggtgcct aatgagtgag ctaactcaca 6960
   ttaattgcgt tgcgctcact gcccgctttc cagtcgggaa acctgtcgtg ccagctgcat 7020
   taatgaatcg gccaacgcgc ggggagaggc ggtttgcgta ttgggcgc 7068
<210> 8
   <211> 2764
   <212> DNA
   <213> Artificial Sequence
<220>
   <223> Predicted pGT232 from PCR of pNCKH103
<400> 8
   tagctgagtc gacaacagtt gttaatcatg acggcttttc taaggggtta gggcttcaag      60
   tactcaaccg gaaaaccgtg tagttccttt tatggcgccg tttatcgaag acttcggggg     120
   ctagaaaatg aaatcaagtt cgatttattt tgaccgattt ttttctcttt cgtggcgatt     180
   ttttaattgg ctggaactta ttttcttagc ttttctgctt ggctgtgttg gcttgtttgc     240
   cgaaaaaatt aatgtttttt tcagcccaaa atttcctcaa tatttcaata tttgggccga     300
   tttctggcag gctggagaaa aattaatcgg atgtagcaag aagttagtta tcgttttatt     360
   ggttgattat tttgtgattt ttttggttgc aaaaatagtt ggaataaaaa ttattcggtc     420
   tagtcgttta tctagcaacc tgcgtcaaat gctgttaaca acagtcgatc ggataccatc     480
   gaccgatcaa aataaacgag ataaagcgaa aaatattagt gcggaaaagg ctaataaagc     540
   cgtcagaaaa tcttttgtga ttgctaagtg ggactcaatt ttggctgtgg taaaaattcc     600
   aaagcagata gaaactagaa aattgctgat tgattatctc aatgatgtcg ctgatgattt     660
   gagtggtcaa actggaatga cttcctcggc gtggcagacg tttctaacgg actaactttt     720
   tctaattatc gagttatgga attcagaaaa taaaaaagca ttgaagcagt gcgctcgctt     780
   tgtcgcaaga caaggagcgg agcgcactgc ttcaatgcat attctgatta atcttgttgc     840
   cgtgttgcag atagtagact ggtgcgaagc ccgctgttag taggtgagtg tcaagcgaca 900
   caacgcctgg agcgtgagcg gaagggcagc gcttgacatg gtgagacggg aagaccctag 960
   ttgagcgaaa gcgagaaaaa agtcattttt tccgctttag cccgcccggc gaggggaaat 1020
   gcggtccagc tgggtcaggg ttttaggggg tgaaacatcc ttagaaactc gctgtaagcg 1080
   agtttcttct tatcttgata catatagaaa caacgccatt ttttgaaatt gggttaaacc 1140
   cttgatatga ctgggtttat taggatttta aaatataaca ctttttggtg tataataagc 1200
   attgtaagaa ttaaataaaa aactcatgca gttgttcgat ttggccgtcg atgcatcagt 1260
   tagttatcgt tattaacttc gaaccctatt ataatatgtc attaggaata tatcaatgat 1320
   aactaactct gactgcttgt agtttttgca gttagaggta taagaaatgt ataaaaaaga 1380
   aattctaaaa gatacctcta gtaatgggaa agttagaccg tggaagcaga aaaaacttgc 1440
   taacttaact tattcccaat atctggaggt tttaaatttt aaaaaagctc ataacgtttc 1500
   aaagtgcgga gaagttcttc aatttgcgaa aactgataat ggtttaaagt tatatcaaac 1560
   gtggttttgt cattcgagac tttgtccatt atgtagctgg cgccgatcga tgaaaaattc 1620
   ttatgagttg cgtcaaattt tagatacagc atataaaaag gatcctagtg ctatatttct 1680
   cttcttgaca ttaactgagg aaagctcaaa gttgggcgag cttagaaaaa atcttacttc 1740
   gatgaatcgt tcaatttatc ggctttttca atataaagat gtaaaaaaag atcttcttgg 1800
   ttatgtgcga tctacagaaa taacggtcaa tcgagatagt ctaactttcc atcaacatgt 1860
   acatattttg ttaatggtga aatctagtta tttttctaag ggtcattatc ttacccaaga 1920
   gaattggtca caattatgga agcgagcaag gaaattagac tacaagccga ttgttaatat 1980
   caagaaaatt cgggcgtcaa aaaaagataa tagtttgatt gcttcggcaa aagaagtaag 2040
   taaatatcaa gttaaagatt atgattatat tacggatgat gagaaaggcg atttagttgt 2100
   tgttgacgag ctcgagcatg ctctggctgg aactcgacaa ttaagctttg cgggattact 2160
   aaaagagatt catcatgagt tattgctgga cgaaaaagaa gatgatctaa ttaatgtgga 2220
   tgacgaaaaa gaaaaagatg atgttgtaga tactgtgata tatcgttgga attctaaggt 2280
   aagtaactat gtaagatggg agtgatagga atggagttac aagatattaa gacaattgac 2340
   aatatgaaga aacaagctga aatctatgga gttatgccag aacagattgc tgataatctt 2400
   caagatttga aaaaattggt tgctgaaaag aaaaatattg atatttatgg cgaactttta 2460
   gatagcttgg ataaagctca acatgatcta tatcgagctt ctattgagtt ggctaatgat 2520
   gaagattgat gctataattc ttatagagaa gtaaaaccgt aatggttgca aaaaagccac 2580
   ccaactaggc ccatagttga gtggctttta atttgcgctt catgccgtta gattattttc 2640
   tccgacgaag ccaatcggag aaaagtgcag tagttaatga gacgattaag ctcaaaactg 2700
   cggagagaat aaatcctcca tggtttctta gacggagaga ataaatcctc catggtttct 2760
   taga 2764
<210> 9
   <211> 4356
   <212> DNA
   <213> Artificial Sequence
<220>
   <223> pSLER1 sequence
<400> 9
   tcttccgctt cctcgctcac tgactcgctg cgctcggtcg ttcggctgcg gcgagcggta      60
   tcagctcact caaaggcggt aatacggtta tccacagaat caggggataa cgcaggaaag     120
   aacatgtgag caaaaggcca gcaaaaggcc aggaaccgta aaaaggccgc gttgctggcg     180
   tttttccata ggctccgccc ccctgacgag catcacaaaa atcgacgctc aagtcagagg     240
   tggcgaaacc cgacaggact ataaagatac caggcgtttc cccctggaag ctccctcgtg     300
   cgctctcctg ttccgaccct gccgcttacc ggatacctgt ccgcctttct cccttcggga     360
   agcgtggcgc tttctcatag ctcacgctgt aggtatctca gttcggtgta ggtcgttcgc     420
   tccaagctgg gctgtgtgca cgaacccccc gttcagcccg accgctgcgc cttatccggt     480
   aactatcgtc ttgagtccaa cccggtaaga cacgacttat cgccactggc agcagccact     540
   ggtaacagga ttagcagagc gaggtatgta ggcggtgcta cagagttctt gaagtggtgg     600
   cctaactacg gctacactag aagaacagta tttggtatct gcgctctgct gaagccagtt     660
   accttcggaa aaagagttgg tagctcttga tccggcaaac aaaccaccgc tggtagcggt     720
   ggtttttttg tttgcaagca gcagattacg cgcagaaaaa aaggatctca agaagatcct     780
   ttgatctttt ctacggggtc tgacgctcag tggaacgaaa actcacgtta agggattttg     840
   gtcatgagat tatcaaaaag gatcttcacc tagatccttt taaattaaaa atgaagtttt     900
   aaatcaatct aaagtatata tgagtaaact tggtctgaca gttaccaatg cttaatcagt     960
   gaggcaccta tctcagcgat ctgtctattt cgttcatcca tagttgcctg actccccgtc    1020
   gtgtagataa ctacgatacg ggagggctta ccatctggcc ccagtgctgc aatgataccg    1080
   cgagacccac gctcaccggc tccagattta tcagcaataa accagccagc cggaagggcc    1140
   gagcgcagaa gtggtcctgc aactttatcc gcctccatcc agtctattaa ttgttgccgg    1200
   gaagctagag taagtagttc gccagttaat agtttgcgca acgttgttgc cattgctaca    1260
   ggcatcgtgg tgtcacgctc gtcgtttggt atggcttcat tcagctccgg ttcccaacga    1320
   tcaaggcgag ttacatgatc ccccatgttg tgcaaaaaag cggttagctc cttcggtcct    1380
   ccgatcgttg tcagaagtaa gttggccgca gtgttatcac tcatggttat ggcagcactg    1440
   cataattctc ttactgtcat gccatccgta agatgctttt ctgtgactgg tgagtactca    1500
   accaagtcat tctgagaata gtgtatgcgg cgaccgagtt gctcttgccc ggcgtcaata 1560
   cgggataata ccgcgccaca tagcagaact ttaaaagtgc tcatcattgg aaaacgttct 1620
   tcggggcgaa aactctcaag gatcttaccg ctgttgagat ccagttcgat gtaacccact 1680
   cgtgcaccca actgatcttc agcatctttt actttcacca gcgtttctgg gtgagcaaaa 1740
   acaggaaggc aaaatgccgc aaaaaaggga ataagggcga cacggaaatg ttgaatactc 1800
   atactcttcc tttttcaata ttattgaagc atttatcagg gttattgtct catgagcgga 1860
   tacatatttg aatgtattta gaaaaataaa caaatagggg ttccgcgcac atttccccga 1920
   aaagtgccac ctgacgtcta agaaaccatt attatcatga cattaaccta taaaaatagg 1980
   cgtatcacga ggccctttcg tctcgcgcgt ttcggtgatg acggtgaaaa cctctgacac 2040
   atgcagctcc cggagacggt cacagcttgt ctgtaagcgg atgccgggag cagacaagcc 2100
   cgtcagggcg cgtcagcggg tgttggcggg tgtcggggct ggcttaacta tgcggcatca 2160
   gagcagattg tactgagagt gcaccataaa attgtaaacg ttaatatttt gttaaaattc 2220
   gcgttaaatt tttgttaaat cagctcattt tttaaccaat aggccgaaat cggcaaaatc 2280
   ccttataaat caaaagaata gcccgagata gggttgagtg ttgttccagt ttggaacaag 2340
   agtccactat taaagaacgt ggactccaac gtcaaagggc gaaaaaccgt ctatcagggc 2400
   gatggcccac tacgtgaacc atcacccaaa tcaagttttt tggggtcgag gtgccgtaaa 2460
   gcactaaatc ggaaccctaa agggagcccc cgatttagag cttgacgggg aaagccggcg 2520
   aacgtggcga gaaaggaagg gaagaaagcg aaaggagcgg gcgctagggc gctggcaagt 2580
   gtagcggtca cgctgcgcgt aaccaccaca cccgccgcgc ttaatgcgcc gctacagggc 2640
   gcgtactatg gttgctttga cgtatgcggt gtgaaatacc gcacagatgc gtaaggagaa 2700
   aataccgcat caggcgccat tcgccattca ggctgcgcaa ctgttgggaa gggcgatcgg 2760
   tgcgggcctc ttcgctatta cgccagctgg cgaaaggggg atgtgctgca aggcgattaa 2820
   gttgggtaac gccagggttt tcccagtcac gacgttgtaa aacgacggcc agtgaattcg 2880
   aatggccatg ggacgtcgac ctgaggtaat tataacccgg gccctatata tggatccaat 2940
   tgcaatgatc atcatgacag atctgcgcgc gatcgatatc agcgctttaa atttgcgcat 3000
   gctagctata gttctagagg taccggttgt taacgttagc cggctacgta tactccggaa 3060
   tattaatagg cctaggatgc atatgttaag agtgtgttga tagtgcagta tcttaaaatt 3120
   ttgtataata ggaattgaag ttaaattaga tgctaaaaat ttgtaattaa gaaggagtga 3180
   ttacatgaac aaaaatataa aatattctca aaacttttta acgagtgaaa aagtactcaa 3240
   ccaaataata aaacaattga atttaaaaga aaccgatacc gtttacgaaa ttggaacagg 3300
   taaagggcat ttaacgacga aactggctaa aataagtaaa caggtaacgt ctattgaatt 3360
   agacagtcat ctattcaact tatcgtcaga aaaattaaaa ctgaatactc gtgtcacttt 3420
   aattcaccaa gatattctac agtttcaatt ccctaacaaa cagaggtata aaattgttgg     3480
   gagtattcct taccatttaa gcacacaaat tattaaaaaa gtggtttttg aaagccatgc     3540
   gtctgacatc tatctgattg ttgaagaagg attctacaag cgtaccttgg atattcaccg     3600
   aacactaggg ttgctcttgc acactcaagt ctcgattcag caattgctta agctgccagc     3660
   ggaatgcttt catcctaaac caaaagtaaa cagtgtctta ataaaactta cccgccatac     3720
   cacagatgtt ccagataaat attggaagct atatacgtac tttgtttcaa aatgggtcaa     3780
   tcgagaatat cgtcaactgt ttactaaaaa tcagtttcat caagcaatga aacacgccaa     3840
   agtaaacaat ttaagtaccg ttacttatga gcaagtattg tctattttta atagttatct     3900
   attatttaac gggaggaaat aattctatga gtcgcttttg taaatttgga aagttacacg     3960
   ttactaaagg gaatgtagat aaattattag gtatactact gacagcttcc aagcatatgg     4020
   cggccgcctg cagctggcgc catcgatacg cgtacgtcgc gaccgcggac atgtacagag     4080
   ctcgagaagt actagtggcc acgtgggccg tgcaccttaa gcttggcgta atcatggtca     4140
   tagctgtttc ctgtgtgaaa ttgttatccg ctcacaattc cacacaacat acgagccgga     4200
   agcataaagt gtaaagcctg gggtgcctaa tgagtgagct aactcacatt aattgcgttg     4260
   cgctcactgc ccgctttcca gtcgggaaac ctgtcgtgcc agctgcatta atgaatcggc     4320
   caacgcgcgg ggagaggcgg tttgcgtatt gggcgc                               4356
<210> 10
   <211> 25
   <212> DNA
   <213> Artificial Sequence
<220>
   <223> Forward primer pGT232 amplification reaction.
<400> 10
   tagctgagtc gacaacagtt gttaa 25
<210> 11
   <211> 30
   <212> DNA
   <213> Artificial Sequence
<220>
   <223> Reverse primer pGT232 amplification reaction.
<400> 11
   tctaagaaac catggaggat ttattctctc 30
<210> 12
   <211> 3054
   <212> DNA
   <213> Artificial Sequence
<220>
   <223> Sequenced pGT232 inserted into pSLER1 for pSLERGT
<400> 12
   ccatggagga tttattctct ccgcagtttt gagcttaatc gtctcattaa ctactgcact      60
   tttctccgat tggcttcgtc ggagaaaata atctaacggc atgaagcgca aattaaaagc     120
   cactcaacta tgggcctagt tgggtggctt ttttgcaacc attacggttt tacttctcta     180
   taagaattat agcatcaatc ttcatcatta gccaactcaa tagaagctcg atatagatca     240
   tgttgagctt tatccaagct atctaaaagt tcgccataaa tatcaatatt tttcttttca     300
   gcaaccaatt ttttcaaatc ttgaagatta tcagcaatct gttctgccat aactccatag     360
   atttcagctt gtttcttcat attgtcaatt gtcttaatat cttgtaactc cattcctatc     420
   actcccatct tacatagtta cttaccttag aattccaacg atatatcaca gtatctacaa     480
   catcatcttt ttctttttcg tcatccacat taattagatc atcttctttt tcgtccagca     540
   ataactcatg atgaatctct tttagtaatc ccgcaaagct taattgtcga gttccagcca     600
   gagcatgctc gagctcgtca acaacaacta aatcgccttt ctcatcatcc gtaatataat     660
   cataatcttt aacttgatat ttacttactt cttttgccga agcaatcaaa ctattatctt     720
   tttttgacgc ccgaattttc ttgatattaa caatcggctt gtagtctaat ttccttgctc     780
   gcttccataa ttgtgaccaa ttctcttggg taagataatg acccttagaa aaataactag     840
   atttcaccat taacaaaata tgtacatgtt gatggaaagt tagactatct cgattgaccg     900
   ttatttctgt agatcgcaca taaccaagaa gatctttttt tacatcttta tattgaaaaa     960
   gccgataaat tgaacgattc atcgaagtaa gattttttct aagctcgccc aactttgagc    1020
   tttcctcagt taatgtcaag aacagaaata tagcactagg atccttttta tatgctgtat    1080
   ctaaaatttg acgcaactca taagaatttt tcatcgatcg gcgccagcta cataatggac    1140
   aaagtctcga atgacaaaac cacgtttgat ataactttaa accattatca gttttcgcaa    1200
   attgaagaac ttctccgcac tttgaaacgt tatgagcttt tttaaaattt aaaacctcca    1260
   gatattggga ataagttaag ttagcaagtt ttttctgctt ccacggtcta actttcccat    1320
   tactagaggt atcttttaga atttcttttt tatacatttc ttatacctct aactgcaaaa    1380
   actacaagca gtcagagtta gttatcattg atatattcct aatgacatat tataataggg    1440
   ttcgaagtta ataacgataa ctaactcatg catcgacggc caaatcgaac aactgcatga    1500
   gttttttatt taattcttac aatgcttatt atacaccaaa aagtgttata ttttaaaatc    1560
   ctaataaacc cagtcatatc aagggtttaa cccaatttca aaaaatggcg ttgtttctat    1620
   atgtatcaag ataagaagaa actcgcttac agcgagtttc taaatagggg tttcacccct    1680
   aaaaccctga ccagctgagc cagctggacc gcatttcccc tcgccgggcg ggcgtaaagc    1740
   ggaaaaaatg acttttttct cgctttcgct caactagggt cttcccgtct caccatgtca    1800
   agcgctgccc ttccgctacg ctccaggcgt ttgtcgcttg acactcacct actaacagcg 1860
   ggcttcgcca ccagtctact atctgcaaca cggcaacaag attaatcaga atatgcattg 1920
   aagcagtgcg ctccgctcct tgtcttgcga caaagcgagc gcactgcttc aatgcttttt 1980
   tattttctga attccataac tcgataatta gaaaaagtta gtccgttaga aacgtctgcc 2040
   acgccgagga agtcattcca gtttgaccac tcaaatcatc agcgacatca ttgagataat 2100
   caatcagcaa ttttctagtt tctatctgct ttggaatttt taccacagcc aaaattgagt 2160
   cccacttagc aatcacaaaa gattttctga cggctttatt agccttttcc gcactaatat 2220
   ttttcgcttt atctcgttta ttttgatcgg tcgatggtat ccgatcgact gttgttacag 2280
   catttgacgc aggttgctag ataaacgact agaccgaata atttttattc caactatttt 2340
   tgcaaccaaa aaaatcacaa aataatcaac caataaaacg ataactaact tcttgctaca 2400
   tccgattaat ttttctccag cctgccagaa atcggcccaa atattgaaat attgaggaaa 2460
   ttttgggctg aaaaaaacat taattttttc ggcaaacaag ccaacacagc caagcagaaa 2520
   agctaagaaa ataagttcca gccaattaaa aaatcgccac gaaagagaaa aaaatcggtc 2580
   aaaataaatc gaacttgatt tcattttcta gcccccgaag tcttcgataa acggcgccat 2640
   aaaaggaact acacggtttt ccggttgagt acttgcaagc cctaacccct tagaaaagcc 2700
   gtcatgatta acaactgttg tcgactcagc tagggcccta tatatggatc caattgcaat 2760
   gatcatcatg acagatctgc gcgcgatcga tatcagcgct ttaaatttgc gcatgctagc 2820
   tatagttcta gaggtaccgg ttgttaacgt tagccggcta cgtatactcc ggaatattaa 2880
   taggcctagg atgcatatgc ttagaagcaa acttaagagt gtgttgatag tgcagtatct 2940
   taaaattttg tataatagga attgaagtta aattagatgc taaaaatttg taattaagaa 3000
   ggagtgatta catgaacaaa aatataaaat attctcaaaa ctttttaacg agtg 3054
<210> 13
   <211> 9013
   <212> DNA
   <213> Artificial Sequence
<220>
   <223> pHENOMM
<400> 13
   tcttccgctt cctcgctcac tgactcgctg cgctcggtcg ttcggctgcg gcgagcggta      60
   tcagctcact caaaggcggt aatacggtta tccacagaat caggggataa cgcaggaaag     120
   aacatgtgag caaaaggcca gcaaaaggcc aggaaccgta aaaaggccgc gttgctggcg     180
   tttttccata ggctccgccc ccctgacgag catcacaaaa atcgacgctc aagtcagagg     240
   tggcgaaacc cgacaggact ataaagatac caggcgtttc cccctggaag ctccctcgtg     300
   cgctctcctg ttccgaccct gccgcttacc ggatacctgt ccgcctttct cccttcggga      360
   agcgtggcgc tttctcatag ctcacgctgt aggtatctca gttcggtgta ggtcgttcgc      420
   tccaagctgg gctgtgtgca cgaacccccc gttcagcccg accgctgcgc cttatccggt      480
   aactatcgtc ttgagtccaa cccggtaaga cacgacttat cgccactggc agcagccact      540
   ggtaacagga ttagcagagc gaggtatgta ggcggtgcta cagagttctt gaagtggtgg      600
   cctaactacg gctacactag aagaacagta tttggtatct gcgctctgct gaagccagtt      660
   accttcggaa aaagagttgg tagctcttga tccggcaaac aaaccaccgc tggtagcggt      720
   ggtttttttg tttgcaagca gcagattacg cgcagaaaaa aaggatctca agaagatcct      780
   ttgatctttt ctacggggtc tgacgctcag tggaacgaaa actcacgtta agggattttg      840
   gtcatgagat tatcaaaaag gatcttcacc tagatccttt taaattaaaa atgaagtttt      900
   aaatcaatct aaagtatata tgagtaaact tggtctgaca gttaccaatg cttaatcagt      960
   gaggcaccta tctcagcgat ctgtctattt cgttcatcca tagttgcctg actccccgtc     1020
   gtgtagataa ctacgatacg ggagggctta ccatctggcc ccagtgctgc aatgataccg     1080
   cgagacccac gctcaccggc tccagattta tcagcaataa accagccagc cggaagggcc     1140
   gagcgcagaa gtggtcctgc aactttatcc gcctccatcc agtctattaa ttgttgccgg     1200
   gaagctagag taagtagttc gccagttaat agtttgcgca acgttgttgc cattgctaca     1260
   ggcatcgtgg tgtcacgctc gtcgtttggt atggcttcat tcagctccgg ttcccaacga     1320
   tcaaggcgag ttacatgatc ccccatgttg tgcaaaaaag cggttagctc cttcggtcct     1380
   ccgatcgttg tcagaagtaa gttggccgca gtgttatcac tcatggttat ggcagcactg     1440
   cataattctc ttactgtcat gccatccgta agatgctttt ctgtgactgg tgagtactca     1500
   accaagtcat tctgagaata gtgtatgcgg cgaccgagtt gctcttgccc ggcgtcaata     1560
   cgggataata ccgcgccaca tagcagaact ttaaaagtgc tcatcattgg aaaacgttct     1620
   tcggggcgaa aactctcaag gatcttaccg ctgttgagat ccagttcgat gtaacccact     1680
   cgtgcaccca actgatcttc agcatctttt actttcacca gcgtttctgg gtgagcaaaa     1740
   acaggaaggc aaaatgccgc aaaaaaggga ataagggcga cacggaaatg ttgaatactc     1800
   atactcttcc tttttcaata ttattgaagc atttatcagg gttattgtct catgagcgga     1860
   tacatatttg aatgtattta gaaaaataaa caaatagggg ttccgcgcac atttccccga     1920
   aaagtgccac ctgacgtcta agaaaccatt attatcatga cattaaccta taaaaatagg     1980
   cgtatcacga ggccctttcg tctcgcgcgt ttcggtgatg acggtgaaaa cctctgacac     2040
   atgcagctcc cggagacggt cacagcttgt ctgtaagcgg atgccgggag cagacaagcc     2100
   cgtcagggcg cgtcagcggg tgttggcggg tgtcggggct ggcttaacta tgcggcatca     2160
   gagcagattg tactgagagt gcaccataaa attgtaaacg ttaatatttt gttaaaattc     2220
   gcgttaaatt tttgttaaat cagctcattt tttaaccaat aggccgaaat cggcaaaatc 2280
   ccttataaat caaaagaata gcccgagata gggttgagtg ttgttccagt ttggaacaag 2340
   agtccactat taaagaacgt ggactccaac gtcaaagggc gaaaaaccgt ctatcagggc 2400
   gatggcccac tacgtgaacc atcacccaaa tcaagttttt tggggtcgag gtgccgtaaa 2460
   gcactaaatc ggaaccctaa agggagcccc cgatttagag cttgacgggg aaagccggcg 2520
   aacgtggcga gaaaggaagg gaagaaagcg aaaggagcgg gcgctagggc gctggcaagt 2580
   gtagcggtca cgctgcgcgt aaccaccaca cccgccgcgc ttaatgcgcc gctacagggc 2640
   gcgtactatg gttgctttga cgtatgcggt gtgaaatacc gcacagatgc gtaaggagaa 2700
   aataccgcat caggcgccat tcgccattca ggctgcgcaa ctgttgggaa gggcgatcgg 2760
   tgcgggcctc ttcgctatta cgccagctgg cgaaaggggg atgtgctgca aggcgattaa 2820
   gttgggtaac gccagggttt tcccagtcac gacgttgtaa aacgacggcc agtgaattcg 2880
   aatggccatg gaggatttat tctctccgca gttttgagct taatcgtctc attaactact 2940
   gcacttttct ccgattggct tcgtcggaga aaataatcta acggcatgaa gcgcaaatta 3000
   aaagccactc aactatgggc ctagttgggt ggcttttttg caaccattac ggttttactt 3060
   ctctataaga attatagcat caatcttcat cattagccaa ctcaatagaa gctcgatata 3120
   gatcatgttg agctttatcc aagctatcta aaagttcgcc ataaatatca atatttttct 3180
   tttcagcaac caattttttc aaatcttgaa gattatcagc aatctgttct gccataactc 3240
   catagatttc agcttgtttc ttcatattgt caattgtctt aatatcttgt aactccattc 3300
   ctatcactcc catcttacat agttacttac cttagaattc caacgatata tcacagtatc 3360
   tacaacatca tctttttctt tttcgtcatc cacattaatt agatcatctt ctttttcgtc 3420
   cagcaataac tcatgatgaa tctcttttag taatcccgca aagcttaatt gtcgagttcc 3480
   agccagagca tgctcgagct cgtcaacaac aactaaatcg cctttctcat catccgtaat 3540
   ataatcataa tctttaactt gatatttact tacttctttt gccgaagcaa tcaaactatt 3600
   atcttttttt gacgcccgaa ttttcttgat attaacaatc ggcttgtagt ctaatttcct 3660
   tgctcgcttc cataattgtg accaattctc ttgggtaaga taatgaccct tagaaaaata 3720
   actagatttc accattaaca aaatatgtac atgttgatgg aaagttagac tatctcgatt 3780
   gaccgttatt tctgtagatc gcacataacc aagaagatct ttttttacat ctttatattg 3840
   aaaaagccga taaattgaac gattcatcga agtaagattt tttctaagct cgcccaactt 3900
   tgagctttcc tcagttaatg tcaagaacag aaatatagca ctaggatcct ttttatatgc 3960
   tgtatctaaa atttgacgca actcataaga atttttcatc gatcggcgcc agctacataa 4020
   tggacaaagt ctcgaatgac aaaaccacgt ttgatataac tttaaaccat tatcagtttt 4080
   cgcaaattga agaacttctc cgcactttga aacgttatga gcttttttaa aatttaaaac 4140
   ctccagatat tgggaataag ttaagttagc aagttttttc tgcttccacg gtctaacttt 4200
   cccattacta gaggtatctt ttagaatttc ttttttatac atttcttata cctctaactg 4260
   caaaaactac aagcagtcag agttagttat cattgatata ttcctaatga catattataa 4320
   tagggttcga agttaataac gataactaac tcatgcatcg acggccaaat cgaacaactg 4380
   catgagtttt ttatttaatt cttacaatgc ttattataca ccaaaaagtg ttatatttta 4440
   aaatcctaat aaacccagtc atatcaaggg tttaacccaa tttcaaaaaa tggcgttgtt 4500
   tctatatgta tcaagataag aagaaactcg cttacagcga gtttctaaat aggggtttca 4560
   cccctaaaac cctgaccagc tgagccagct ggaccgcatt tcccctcgcc gggcgggcgt 4620
   aaagcggaaa aaatgacttt tttctcgctt tcgctcaact agggtcttcc cgtctcacca 4680
   tgtcaagcgc tgcccttccg ctacgctcca ggcgtttgtc gcttgacact cacctactaa 4740
   cagcgggctt cgccaccagt ctactatctg caacacggca acaagattaa tcagaatatg 4800
   cattgaagca gtgcgctccg ctccttgtct tgcgacaaag cgagcgcact gcttcaatgc 4860
   ttttttattt tctgaattcc ataactcgat aattagaaaa agttagtccg ttagaaacgt 4920
   ctgccacgcc gaggaagtca ttccagtttg accactcaaa tcatcagcga catcattgag 4980
   ataatcaatc agcaattttc tagtttctat ctgctttgga atttttacca cagccaaaat 5040
   tgagtcccac ttagcaatca caaaagattt tctgacggct ttattagcct tttccgcact 5100
   aatatttttc gctttatctc gtttattttg atcggtcgat ggtatccgat cgactgttgt 5160
   tacagcattt gacgcaggtt gctagataaa cgactagacc gaataatttt tattccaact 5220
   atttttgcaa ccaaaaaaat cacaaaataa tcaaccaata aaacgataac taacttcttg 5280
   ctacatccga ttaatttttc tccagcctgc cagaaatcgg cccaaatatt gaaatattga 5340
   ggaaattttg ggctgaaaaa aacattaatt ttttcggcaa acaagccaac acagccaagc 5400
   agaaaagcta agaaaataag ttccagccaa ttaaaaaatc gccacgaaag agaaaaaaat 5460
   cggtcaaaat aaatcgaact tgatttcatt ttctagcccc cgaagtcttc gataaacggc 5520
   gccataaaag gaactacacg gttttccggt tgagtacttg caagccctaa ccccttagaa 5580
   aagccgtcat gattaacaac tgttgtcgac ttttttttga taagttaagg gatgcagttt 5640
   atgcacccct taacttatca ttattagtgt aaacaaggaa ggatatcttg tacggcttga 5700
   acaattacac caccagcagc gatatcagca gaaatacgag caatatgttc gtcaagtcct 5760
   tgttcgttat cgttccaaat atatggccga tctgatgtag gtttttggcc gacaacatga 5820
   cgtactgctg aataaagacg ttctgtagct ggacttaaac aagcacgagc atcataatgg 5880
   cccgtctttt tgtaagtccg taaatcaaca gcttgtactc caaacattaa tgcaatagct 5940
   acgtagtttt gaaaaatatc aacagaccgt cgtgctaacg tagcagaagt gtatccttga 6000
   gagttaatat tttggttaaa ttgttcggcg tgtgtaggaa agcgatcagc aattgaattt 6060
   ccgtaaaaag tcaacaatgg cataattgag ttgccacaaa tttgcaaacc cttaagaccc 6120
   atgtttacct ttcgttcccg gtttcccaat aatgatggtg gaagtccgtt actgaattct 6180
   ggactggcca aaagtgcaat ttggacatcc aaatgctttg caagaagacc aatgtagtaa 6240
   cgtaagtggt ccatgcccat gccaacatat tgacccaaaa agttaccgcc atggtatgag 6300
   gcttggttat caacgtcaat taatggattg tcagtgacac tattaatttc gatttcaatt 6360
   tgcttagcaa tttgtgaaat tccatcaaca ataggaccca aatattgtgg taagcaacgt 6420
   aaactatagc ggtcttgaat aagttcatga tcacgataat catgcttacc atctaattcg 6480
   tcgcgaacaa gttgactatt agctaaaaga gaaatcattt gatcagcagc ccaaagttga 6540
   cctgggtgag gtttagaatt gtgaatgaat gggtggaaac tttgatttgt gccattaagt 6600
   gcttgaatat ctaaggcatg aacacccatg gcaatagcag taagaatttg agtatcataa 6660
   acgcaattag cagcaattcc cgtcataact gatgtaccat tcatcatagc aagaccttct 6720
   tttggaagta atgtaagtgg tgataaattt aattgtcgaa gagctgttgg ggcatccatt 6780
   tctttaccat taaaatcaac tttgaatgat ggatccaaac caattaaaga acctgtaata 6840
   taagataaag gaacaagatc tccacttgca ccaattgatc cgaattcgta aacataaggt 6900
   gtaacaccgg cgttaaggaa aatttccata cgcttaatta attcaagacg aataccagaa 6960
   gcgccacgca tgtgtgaatt tgcacgtaat aacatagcag cacgaacatc tgctaatgga 7020
   agtttattac cagctcctgt tttaagaaac caaacaagat tagtttgaag ttcagaagct 7080
   tgttcacgac taatagcgac gtttgccatt ccaccgaatc ctgaagtaac accataaatt 7140
   ggttctcctg attcaacagc gttattgata tagtcgcaac tagcttgaat gccttgtaaa 7200
   atgtcagtgt tgttagttaa actaactaaa gtaccattgc gtgcaacccg tgcaacatcg 7260
   ttaatagtaa gcttttgatt accaatgata acattagcac ttgagtttcc agtaaatgag 7320
   aattgttgag aactagtctt agattgtgct tgagataaag tcttcattta tacctactcc 7380
   ttcttaatta caaattttta gcatctaatt taacttcaat tcctattata caaaatttta 7440
   agatactgca ctatcaacac actcttgaac atatgcatcc taggtcgtct attaatactt 7500
   ccaggagtat acgtagccag gctaacgtta atcaaccagg tatcctctag aactatagct 7560
   agcatgccta tatatggatc caattgcaat gatcatcatg acagatctgc gcgcgatcga 7620
   tatcagcgct ttaaatttgc gcatgctagc tatagttcta gaggtaccgg ttgttaacgt 7680
   tagccggcta cgtatactcc ggaatattaa taggcctagg atgcatatgc ttagaagcaa 7740
   acttaagagt gtgttgatag tgcagtatct taaaattttg tataatagga attgaagtta 7800
   aattagatgc taaaaatttg taattaagaa ggagtgatta catgaacaaa aatataaaat 7860
   attctcaaaa ctttttaacg agtgaaaaag tactcaacca aataataaaa caattgaatt 7920
   taaaagaaac cgataccgtt tacgaaattg gaacaggtaa agggcattta acgacgaaac 7980
   tggctaaaat aagtaaacag gtaacgtcta ttgaattaga cagtcatcta ttcaacttat 8040
   cgtcagaaaa attaaaactg aatactcgtg tcactttaat tcaccaagat attctacagt 8100
   ttcaattccc taacaaacag aggtataaaa ttgttgggag tattccttac catttaagca 8160
   cacaaattat taaaaaagtg gtttttgaaa gccatgcgtc tgacatctat ctgattgttg 8220
   aagaaggatt ctacaagcgt accttggata ttcaccgaac actagggttg ctcttgcaca 8280
   ctcaagtctc gattcagcaa ttgcttaagc tgccagcgga atgctttcat cctaaaccaa 8340
   aagtaaacag tgtcttaata aaacttaccc gccataccac agatgttcca gataaatatt 8400
   ggaagctata tacgtacttt gtttcaaaat gggtcaatcg agaatatcgt caactgttta 8460
   ctaaaaatca gtttcatcaa gcaatgaaac acgccaaagt aaacaattta agtaccgtta 8520
   cttatgagca agtattgtct atttttaata gttatctatt atttaacggg aggaaataat 8580
   tctatgagtc gcttttgtaa atttggaaag ttacacgtta ctaaagggaa tgtagataaa 8640
   ttattaggta tactactgac agcttccaag catatggcgg ccgcctgcag ctggcgccat 8700
   cgatacgcgt acgtcgcgac cgcggacatg tacagagctc gagaagtact agtggccacg 8760
   tgggccgtgc accttaagct tggcgtaatc atggtcatag ctgtttcctg tgtgaaattg 8820
   ttatccgctc acaattccac acaacatacg agccggaagc ataaagtgta aagcctgggg 8880
   tgcctaatga gtgagctaac tcacattaat tgcgttgcgc tcactgcccg ctttccagtc 8940
   gggaaacctg tcgtgccagc tgcattaatg aatcggccaa cgcgcgggga gaggcggttt 9000
   gcgtattggg cgc 9013
<210> 14
   <211> 9142
   <212> DNA
   <213> Artificial Sequence
<220>
   <223> pHENOMM-sec
<400> 14
   tcttccgctt cctcgctcac tgactcgctg cgctcggtcg ttcggctgcg gcgagcggta      60
   tcagctcact caaaggcggt aatacggtta tccacagaat caggggataa cgcaggaaag     120
   aacatgtgag caaaaggcca gcaaaaggcc aggaaccgta aaaaggccgc gttgctggcg     180
   tttttccata ggctccgccc ccctgacgag catcacaaaa atcgacgctc aagtcagagg     240
   tggcgaaacc cgacaggact ataaagatac caggcgtttc cccctggaag ctccctcgtg     300
   cgctctcctg ttccgaccct gccgcttacc ggatacctgt ccgcctttct cccttcggga     360
   agcgtggcgc tttctcatag ctcacgctgt aggtatctca gttcggtgta ggtcgttcgc     420
   tccaagctgg gctgtgtgca cgaacccccc gttcagcccg accgctgcgc cttatccggt 480
   aactatcgtc ttgagtccaa cccggtaaga cacgacttat cgccactggc agcagccact 540
   ggtaacagga ttagcagagc gaggtatgta ggcggtgcta cagagttctt gaagtggtgg 600
   cctaactacg gctacactag aagaacagta tttggtatct gcgctctgct gaagccagtt 660
   accttcggaa aaagagttgg tagctcttga tccggcaaac aaaccaccgc tggtagcggt 720
   ggtttttttg tttgcaagca gcagattacg cgcagaaaaa aaggatctca agaagatcct 780
   ttgatctttt ctacggggtc tgacgctcag tggaacgaaa actcacgtta agggattttg 840
   gtcatgagat tatcaaaaag gatcttcacc tagatccttt taaattaaaa atgaagtttt 900
   aaatcaatct aaagtatata tgagtaaact tggtctgaca gttaccaatg cttaatcagt 960
   gaggcaccta tctcagcgat ctgtctattt cgttcatcca tagttgcctg actccccgtc 1020
   gtgtagataa ctacgatacg ggagggctta ccatctggcc ccagtgctgc aatgataccg 1080
   cgagacccac gctcaccggc tccagattta tcagcaataa accagccagc cggaagggcc 1140
   gagcgcagaa gtggtcctgc aactttatcc gcctccatcc agtctattaa ttgttgccgg 1200
   gaagctagag taagtagttc gccagttaat agtttgcgca acgttgttgc cattgctaca 1260
   ggcatcgtgg tgtcacgctc gtcgtttggt atggcttcat tcagctccgg ttcccaacga 1320
   tcaaggcgag ttacatgatc ccccatgttg tgcaaaaaag cggttagctc cttcggtcct 1380
   ccgatcgttg tcagaagtaa gttggccgca gtgttatcac tcatggttat ggcagcactg 1440
   cataattctc ttactgtcat gccatccgta agatgctttt ctgtgactgg tgagtactca 1500
   accaagtcat tctgagaata gtgtatgcgg cgaccgagtt gctcttgccc ggcgtcaata 1560
   cgggataata ccgcgccaca tagcagaact ttaaaagtgc tcatcattgg aaaacgttct 1620
   tcggggcgaa aactctcaag gatcttaccg ctgttgagat ccagttcgat gtaacccact 1680
   cgtgcaccca actgatcttc agcatctttt actttcacca gcgtttctgg gtgagcaaaa 1740
   acaggaaggc aaaatgccgc aaaaaaggga ataagggcga cacggaaatg ttgaatactc 1800
   atactcttcc tttttcaata ttattgaagc atttatcagg gttattgtct catgagcgga 1860
   tacatatttg aatgtattta gaaaaataaa caaatagggg ttccgcgcac atttccccga 1920
   aaagtgccac ctgacgtcta agaaaccatt attatcatga cattaaccta taaaaatagg 1980
   cgtatcacga ggccctttcg tctcgcgcgt ttcggtgatg acggtgaaaa cctctgacac 2040
   atgcagctcc cggagacggt cacagcttgt ctgtaagcgg atgccgggag cagacaagcc 2100
   cgtcagggcg cgtcagcggg tgttggcggg tgtcggggct ggcttaacta tgcggcatca 2160
   gagcagattg tactgagagt gcaccataaa attgtaaacg ttaatatttt gttaaaattc 2220
   gcgttaaatt tttgttaaat cagctcattt tttaaccaat aggccgaaat cggcaaaatc 2280
   ccttataaat caaaagaata gcccgagata gggttgagtg ttgttccagt ttggaacaag 2340
   agtccactat taaagaacgt ggactccaac gtcaaagggc gaaaaaccgt ctatcagggc 2400
   gatggcccac tacgtgaacc atcacccaaa tcaagttttt tggggtcgag gtgccgtaaa 2460
   gcactaaatc ggaaccctaa agggagcccc cgatttagag cttgacgggg aaagccggcg 2520
   aacgtggcga gaaaggaagg gaagaaagcg aaaggagcgg gcgctagggc gctggcaagt 2580
   gtagcggtca cgctgcgcgt aaccaccaca cccgccgcgc ttaatgcgcc gctacagggc 2640
   gcgtactatg gttgctttga cgtatgcggt gtgaaatacc gcacagatgc gtaaggagaa 2700
   aataccgcat caggcgccat tcgccattca ggctgcgcaa ctgttgggaa gggcgatcgg 2760
   tgcgggcctc ttcgctatta cgccagctgg cgaaaggggg atgtgctgca aggcgattaa 2820
   gttgggtaac gccagggttt tcccagtcac gacgttgtaa aacgacggcc agtgaattcg 2880
   aatggccatg gaggatttat tctctccgca gttttgagct taatcgtctc attaactact 2940
   gcacttttct ccgattggct tcgtcggaga aaataatcta acggcatgaa gcgcaaatta 3000
   aaagccactc aactatgggc ctagttgggt ggcttttttg caaccattac ggttttactt 3060
   ctctataaga attatagcat caatcttcat cattagccaa ctcaatagaa gctcgatata 3120
   gatcatgttg agctttatcc aagctatcta aaagttcgcc ataaatatca atatttttct 3180
   tttcagcaac caattttttc aaatcttgaa gattatcagc aatctgttct gccataactc 3240
   catagatttc agcttgtttc ttcatattgt caattgtctt aatatcttgt aactccattc 3300
   ctatcactcc catcttacat agttacttac cttagaattc caacgatata tcacagtatc 3360
   tacaacatca tctttttctt tttcgtcatc cacattaatt agatcatctt ctttttcgtc 3420
   cagcaataac tcatgatgaa tctcttttag taatcccgca aagcttaatt gtcgagttcc 3480
   agccagagca tgctcgagct cgtcaacaac aactaaatcg cctttctcat catccgtaat 3540
   ataatcataa tctttaactt gatatttact tacttctttt gccgaagcaa tcaaactatt 3600
   atcttttttt gacgcccgaa ttttcttgat attaacaatc ggcttgtagt ctaatttcct 3660
   tgctcgcttc cataattgtg accaattctc ttgggtaaga taatgaccct tagaaaaata 3720
   actagatttc accattaaca aaatatgtac atgttgatgg aaagttagac tatctcgatt 3780
   gaccgttatt tctgtagatc gcacataacc aagaagatct ttttttacat ctttatattg 3840
   aaaaagccga taaattgaac gattcatcga agtaagattt tttctaagct cgcccaactt 3900
   tgagctttcc tcagttaatg tcaagaacag aaatatagca ctaggatcct ttttatatgc 3960
   tgtatctaaa atttgacgca actcataaga atttttcatc gatcggcgcc agctacataa 4020
   tggacaaagt ctcgaatgac aaaaccacgt ttgatataac tttaaaccat tatcagtttt 4080
   cgcaaattga agaacttctc cgcactttga aacgttatga gcttttttaa aatttaaaac 4140
   ctccagatat tgggaataag ttaagttagc aagttttttc tgcttccacg gtctaacttt 4200
   cccattacta gaggtatctt ttagaatttc ttttttatac atttcttata cctctaactg 4260
   caaaaactac aagcagtcag agttagttat cattgatata ttcctaatga catattataa 4320
   tagggttcga agttaataac gataactaac tcatgcatcg acggccaaat cgaacaactg 4380
   catgagtttt ttatttaatt cttacaatgc ttattataca ccaaaaagtg ttatatttta 4440
   aaatcctaat aaacccagtc atatcaaggg tttaacccaa tttcaaaaaa tggcgttgtt 4500
   tctatatgta tcaagataag aagaaactcg cttacagcga gtttctaaat aggggtttca 4560
   cccctaaaac cctgaccagc tgagccagct ggaccgcatt tcccctcgcc gggcgggcgt 4620
   aaagcggaaa aaatgacttt tttctcgctt tcgctcaact agggtcttcc cgtctcacca 4680
   tgtcaagcgc tgcccttccg ctacgctcca ggcgtttgtc gcttgacact cacctactaa 4740
   cagcgggctt cgccaccagt ctactatctg caacacggca acaagattaa tcagaatatg 4800
   cattgaagca gtgcgctccg ctccttgtct tgcgacaaag cgagcgcact gcttcaatgc 4860
   ttttttattt tctgaattcc ataactcgat aattagaaaa agttagtccg ttagaaacgt 4920
   ctgccacgcc gaggaagtca ttccagtttg accactcaaa tcatcagcga catcattgag 4980
   ataatcaatc agcaattttc tagtttctat ctgctttgga atttttacca cagccaaaat 5040
   tgagtcccac ttagcaatca caaaagattt tctgacggct ttattagcct tttccgcact 5100
   aatatttttc gctttatctc gtttattttg atcggtcgat ggtatccgat cgactgttgt 5160
   tacagcattt gacgcaggtt gctagataaa cgactagacc gaataatttt tattccaact 5220
   atttttgcaa ccaaaaaaat cacaaaataa tcaaccaata aaacgataac taacttcttg 5280
   ctacatccga ttaatttttc tccagcctgc cagaaatcgg cccaaatatt gaaatattga 5340
   ggaaattttg ggctgaaaaa aacattaatt ttttcggcaa acaagccaac acagccaagc 5400
   agaaaagcta agaaaataag ttccagccaa ttaaaaaatc gccacgaaag agaaaaaaat 5460
   cggtcaaaat aaatcgaact tgatttcatt ttctagcccc cgaagtcttc gataaacggc 5520
   gccataaaag gaactacacg gttttccggt tgagtacttg caagccctaa ccccttagaa 5580
   aagccgtcat gattaacaac tgttgtcgac ttttttttga taagttaagg gatgcagttt 5640
   atgcacccct taacttatca ttattagtgt aaacaaggaa ggatatcttg tacggcttga 5700
   acaattacac caccagcagc gatatcagca gaaatacgag caatatgttc gtcaagtcct 5760
   tgttcgttat cgttccaaat atatggccga tctgatgtag gtttttggcc gacaacatga 5820
   cgtactgctg aataaagacg ttctgtagct ggacttaaac aagcacgagc atcataatgg 5880
   cccgtctttt tgtaagtccg taaatcaaca gcttgtactc caaacattaa tgcaatagct 5940
   acgtagtttt gaaaaatatc aacagaccgt cgtgctaacg tagcagaagt gtatccttga 6000
   gagttaatat tttggttaaa ttgttcggcg tgtgtaggaa agcgatcagc aattgaattt 6060
   ccgtaaaaag tcaacaatgg cataattgag ttgccacaaa tttgcaaacc cttaagaccc 6120
   atgtttacct ttcgttcccg gtttcccaat aatgatggtg gaagtccgtt actgaattct 6180
   ggactggcca aaagtgcaat ttggacatcc aaatgctttg caagaagacc aatgtagtaa 6240
   cgtaagtggt ccatgcccat gccaacatat tgacccaaaa agttaccgcc atggtatgag 6300
   gcttggttat caacgtcaat taatggattg tcagtgacac tattaatttc gatttcaatt 6360
   tgcttagcaa tttgtgaaat tccatcaaca ataggaccca aatattgtgg taagcaacgt 6420
   aaactatagc ggtcttgaat aagttcatga tcacgataat catgcttacc atctaattcg 6480
   tcgcgaacaa gttgactatt agctaaaaga gaaatcattt gatcagcagc ccaaagttga 6540
   cctgggtgag gtttagaatt gtgaatgaat gggtggaaac tttgatttgt gccattaagt 6600
   gcttgaatat ctaaggcatg aacacccatg gcaatagcag taagaatttg agtatcataa 6660
   acgcaattag cagcaattcc cgtcataact gatgtaccat tcatcatagc aagaccttct 6720
   tttggaagta atgtaagtgg tgataaattt aattgtcgaa gagctgttgg ggcatccatt 6780
   tctttaccat taaaatcaac tttgaatgat ggatccaaac caattaaaga acctgtaata 6840
   taagataaag gaacaagatc tccacttgca ccaattgatc cgaattcgta aacataaggt 6900
   gtaacaccgg cgttaaggaa aatttccata cgcttaatta attcaagacg aataccagaa 6960
   gcgccacgca tgtgtgaatt tgcacgtaat aacatagcag cacgaacatc tgctaatgga 7020
   agtttattac cagctcctgt tttaagaaac caaacaagat tagtttgaag ttcagaagct 7080
   tgttcacgac taatagcgac gtttgccatt ccaccgaatc ctgaagtaac accataaatt 7140
   ggttctcctg attcaacagc gttattgata tagtcgcaac tagcttgaat gccttgtaaa 7200
   atgtcagtgt tgttagttaa actaactaaa gtaccattgc gtgcaacccg tgcaacatcg 7260
   ttaatagtaa gcttttgatt accaatgata acattagcac ttgagtttcc agtaaatgag 7320
   aattgttgag aactagtctt agattgtgct tgagataaag tcttcattta tacctactcc 7380
   tatcaactga agaagcatca acaaccataa tattttggct tacgccccaa aaactaataa 7440
   cgaccaataa tggtaatagc cacacaggaa gtccgttacg gtgatgttta cctgtatttt 7500
   cagtatacat tcttaattac aaatttttag catctaattt aacttcaatt cctattatac 7560
   aaaattttaa gatactgcac tatcaacaca ctcttgaaca tatgcatcct aggtcgtcta 7620
   ttaatacttc caggagtata cgtagccagg ctaacgttaa tcaaccaggt atcctctaga 7680
   actatagcta gcatgcctat atatggatcc aattgcaatg atcatcatga cagatctgcg 7740
   cgcgatcgat atcagcgctt taaatttgcg catgctagct atagttctag aggtaccggt 7800
   tgttaacgtt agccggctac gtatactccg gaatattaat aggcctagga tgcatatgct 7860
   tagaagcaaa cttaagagtg tgttgatagt gcagtatctt aaaattttgt ataataggaa 7920
   ttgaagttaa attagatgct aaaaatttgt aattaagaag gagtgattac atgaacaaaa 7980
   atataaaata ttctcaaaac tttttaacga gtgaaaaagt actcaaccaa ataataaaac 8040
   aattgaattt aaaagaaacc gataccgttt acgaaattgg aacaggtaaa gggcatttaa 8100
   cgacgaaact ggctaaaata agtaaacagg taacgtctat tgaattagac agtcatctat 8160
   tcaacttatc gtcagaaaaa ttaaaactga atactcgtgt cactttaatt caccaagata 8220
   ttctacagtt tcaattccct aacaaacaga ggtataaaat tgttgggagt attccttacc 8280
   atttaagcac acaaattatt aaaaaagtgg tttttgaaag ccatgcgtct gacatctatc 8340
   tgattgttga agaaggattc tacaagcgta ccttggatat tcaccgaaca ctagggttgc 8400
   tcttgcacac tcaagtctcg attcagcaat tgcttaagct gccagcggaa tgctttcatc 8460
   ctaaaccaaa agtaaacagt gtcttaataa aacttacccg ccataccaca gatgttccag 8520
   ataaatattg gaagctatat acgtactttg tttcaaaatg ggtcaatcga gaatatcgtc 8580
   aactgtttac taaaaatcag tttcatcaag caatgaaaca cgccaaagta aacaatttaa 8640
   gtaccgttac ttatgagcaa gtattgtcta tttttaatag ttatctatta tttaacggga 8700
   ggaaataatt ctatgagtcg cttttgtaaa tttggaaagt tacacgttac taaagggaat 8760
   gtagataaat tattaggtat actactgaca gcttccaagc atatggcggc cgcctgcagc 8820
   tggcgccatc gatacgcgta cgtcgcgacc gcggacatgt acagagctcg agaagtacta 8880
   gtggccacgt gggccgtgca ccttaagctt ggcgtaatca tggtcatagc tgtttcctgt 8940
   gtgaaattgt tatccgctca caattccaca caacatacga gccggaagca taaagtgtaa 9000
   agcctggggt gcctaatgag tgagctaact cacattaatt gcgttgcgct cactgcccgc 9060
   tttccagtcg ggaaacctgt cgtgccagct gcattaatga atcggccaac gcgcggggag 9120
   aggcggtttg cgtattgggc gc 9142
<210> 15
   <211> 1963
   <212> DNA
   <213> Artificial Sequence
<220>
   <223> synthesized
<400> 15
   gcatgctagc tatagttcta gaggatacct ggttgattaa cgttagcctg gctacgtata      60
   ctcctggaag tattaataga cgacctagga tgcatatgtt caagagtgtg ttgatagtgc     120
   agtatcttaa aattttgtat aataggaatt gaagttaaat tagatgctaa aaatttgtaa     180
   ttaagaagga gtaggtataa atgaagactt tatctcaagc acaatctaag actagttctc     240
   aacaattctc atttactgga aactcaagtg ctaatgttat cattggtaat caaaagctta     300
   ctattaacga tgttgcacgg gttgcacgca atggtacttt agttagttta actaacaaca     360
   ctgacatttt acaaggcatt caagctagtt gcgactatat caataacgct gttgaatcag      420
   gagaaccaat ttatggtgtt acttcaggat tcggtggaat ggcaaacgtc gctattagtc      480
   gtgaacaagc ttctgaactt caaactaatc ttgtttggtt tcttaaaaca ggagctggta      540
   ataaacttcc attagcagat gttcgtgctg ctatgttatt acgtgcaaat tcacacatgc      600
   gtggcgcttc tggtattcgt cttgaattaa ttaagcgtat ggaaattttc cttaacgccg      660
   gtgttacacc ttatgtttac gaattcggat caattggtgc aagtggagat cttgttcctt      720
   tatcttatat tacaggttct ttaattggtt tggatccatc attcaaagtt gattttaatg      780
   gtaaagaaat ggatgcccca acagctcttc gacaattaaa tttatcacca cttacattac      840
   ttccaaaaga aggtcttgct atgatgaatg gtacatcagt tatgacggga attgctgcta      900
   attgcgttta tgatactcaa attcttactg ctattgccat gggtgttcat gccttagata      960
   ttcaagcact taatggcaca aatcaaagtt tccacccatt cattcacaat tctaaacctc     1020
   acccaggtca actttgggct gctgatcaaa tgatttctct tttagctaat agtcaacttg     1080
   ttcgcgacga attagatggt aagcatgatt atcgtgatca tgaacttatt caagaccgct     1140
   atagtttacg ttgcttacca caatatttgg gtcctattgt tgatggaatt tcacaaattg     1200
   ctaagcaaat tgaaatcgaa attaatagtg tcactgacaa tccattaatt gacgttgata     1260
   accaagcctc ataccatggc ggtaactttt tgggtcaata tgttggcatg ggcatggacc     1320
   acttacgtta ctacattggt cttcttgcaa agcatttgga tgtccaaatt gcacttttgg     1380
   ccagtccaga attcagtaac ggacttccac catcattatt gggaaaccgg gaacgaaagg     1440
   taaacatggg tcttaagggt ttgcaaattt gtggcaactc aattatgcca ttgttgactt     1500
   tttacggaaa ttcaattgct gatcgctttc ctacacacgc cgaacaattt aaccaaaata     1560
   ttaactctca aggatacact tctgctacgt tagcacgacg gtctgttgat atttttcaaa     1620
   actacgtagc tattgcatta atgtttggag tacaagctgt tgatttacgg acttacaaaa     1680
   agacgggcca ttatgatgct cgtgcttgtt taagtccagc tacagaacgt ctttattcag     1740
   cagtacgtca tgttgtcggc caaaaaccta catcagatcg gccatatatt tggaacgata     1800
   acgaacaagg acttgacgaa catattgctc gtatttctgc tgatatcgct gctggtggtg     1860
   taattgttca agccgtacaa gatatccttc cttgtttaca ctaataatga taagttaagg     1920
   ggtgcataaa ctgcatccct taacttatca aaaaaaagtc gac                       1963

## Description

### BACKGROUND

The present invention relates to a genetically modified probiotic (GMP), and more specifically to a GMP adapted to provide the phenylalanine ammonia-lyase (PAL) gene or other phenylalanine-degrading enzyme for the treatment of phenylketonuria (PKU). The GMP is herein referred to as PHEnominal.

### Advantages Over Conventional Technologies

PKU is a disease that renders the patient incapable of digesting the amino acid phenylalanine, resulting in a subsequent accumulation in the blood causing toxicity.

Several technologies have been developed for the treatment of PKU. First, liver and/or hepatocyte transplants have been used. A regular transplant is not considered an acceptable treatment by insurance companies as the cost of treatment for PKU transfers to the cost of treatment to prevent organ rejection.

Second, gene therepy techniques have been utilized for the treatment of PKU. Gene therapy methods have yet to break the barrier of gene silencing. In gene silencing, a eukaryotic cell detects genes of a viral source, such as a gene therapy vector, and methylates the DNA to silence it. Additionally, after the "Bubble boy" syndrome incident (children developing leukemia following gene therapy for a terminal illness), gene therapy has not gained wide spread acceptance or regulatory approval in the USA.

Finally, several other treatments have been tried, without the level of cost-effectiveness required for wide-spread adoption.

Other treatments for PKU patients are made difficult by the many co-factors and co-enzymes required for PAH (the deficient enzyme) to function. Current dietary therapies cost approximately $40,000.00 per year for a teen or adult. Co-factor therapy, which supplies an excess of PAH's co-factor, reduces phenylalanine (phe) in some of the patients. However many patients (40 - 50%) see no improvements, or improvement is not sufficient to warrant the cost of co-factor therapy. The cofactor therapy by the name of Kuvan®, is also very expensive ($40,000.00 per year for an adult or teen). Responses from this treatment range from no change to complete control of phenylalanine levels. It is thought that the patient's level of response to this therapy is dictated by the exact genetic mutation they carry.

PEG-PAL, is currently under clinical trials. For this treatment, PAL is produced by bacteria, purified, and coated in polyethylene glycol (PEG). The resulting PEG-PAL is injected subcutaneously into the patient, and enters the blood stream. Once in the blood, the PEG coating helps protect PAL from immunodetection while allowing the enzyme to degrade serum phenylalanine. Although cost estimates have not been released, similar therapies (PEG-Adenosine deaminase) are quite expensive. Additionally, complications of this therapy have been detected in phase 2 clinical trials.

Yeast PAL in microcapsules has also been studied as a potential treatment. This study used rats injected with phenylalanine rather than a true disease model. Yeast PAL has lower activity (cannot compare exactly, due to notation used), and of further concern, yeast PAL is nearly as good at catabolizing tyrosine as phe (.67 micromoles/min per unit enzyme for tyrosine and 1.4 micromoles/min per unit enzyme at catabolizing phe). The latter is of particular concern since PKU patients also are unable to synthesize the essential amino acid tyrosine from phe. Additionally, microcapsules were deemed too expensive to use as a PKU therapy.

Oral naked PAL enzyme has been developed, but was only used in mice due to the restrictions of treatment. Namely, the PAL enzyme could not survive the stomach to arrive in the intestine unless it was given by gastric gavage along with enough sodium bicarbonate to neutralize the stomach acid. Furthermore, once in the intestine, PAL is cleaved rapidly by proteases such as trypsin and chymotrypsin.

The only current option known to work as a treatment in all patients with PKU is dietary restriction of the ingestion of foods containing phe. No natural protein is free of phe, and only a handful of proteins have been identified as having low phe contents. To this end, restricting phe ingestion can be fostered by the ingestion of synthetic foods. These foods are reported as having repugnant odor and flavor, resulting in poor dietary compliance. In addition to a diet more limited than that of a person with gluten intolerance, and the cost of the synthetic food diet is similar to that of the co-factor therapy ($1,000.00 per month for infant formula, with costs increasing as the patient becomes an adult and requires more food). Insurance companies commonly deny assistance with the food expenses of this treatment.

The invention described herein meets the need for a cost-effective therapy for PKU, with tolerable side-effects and high efficacy.

### SUMMARY

The present invention relates to a GMP as defined in the claims, adapted to provide the PAL gene or other phenylalanine-degrading enzyme for the treatment of PKU. In certain embodiments, the GMP of the present invention comprises a probiotic, a PAL gene to be expressed using the probiotic, wherein the PAL gene is functionally attached to a promoter and a ribosome binding site, and may be codon-optimized for expression in a certain host organism.

The present invention further comprises a GMP as defined in the claims for use in a method of treating the metabolic disease of PKU by oral administration and ingestion of a GMP. The GMP described herein will produce the enzyme PAL in the intestine when administered orally. The genetically engineered PAL will digest ingested phenylalanine contained in most proteinous foods. The end result will be that phenylalanine obtained from ingestion is not absorbed into the blood stream and cannot build up to toxic levels.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
FIGURE 1 shows the creation of the final functional mouse therapy shuttle vector starting with A) the *E. coli* plasmid pSLER1, B) addition of the pGT232 fragment to create shuttle vector ability, and C) final addition of FuzErmAvPAL for AvPAL expression when in a probiotic host; and
FIGURE 2 shows trans-cinnamic acid production by plasmid carried/100-23 cell line in an embodiment of the present invention.

### DETAILED DESCRIPTION OF PREFERRED EMBODIMENTS

One aspect of the present invention pertains to a genetically modified probiotic (GMP) to deliver functional PAL enzyme or other phenylalanine-degrading enzyme to the body of an animal, preferably to the small intestine. In certain embodiments, the probiotic is/has 1) capable of surviving the acid of the stomach and maintaining functionality after exiting the stomach; 2) capable of maintaining functionality and metabolic activity in the small intestine, and expressing the PAL gene or other phenylalanine-degrading enzyme in the intestine; 3) a mouse trophic and human trophic strain; 4) safe for oral use as a human probiotic; 5) capable of being produced in-vitro; and 6) amendable to electropration or another form of transformation.

In some embodiments, the probiotic capable of delivering functional phenylalanine-degrading enzyme, such as PAL enzyme, is useful in the treatment of PKU. If phenylalanine is pulled from the intestinal lumen by probiotics to be degraded by bacterial cells, phe will not enter the blood and will be unable to cause its pathogenic effects in a PKU animal/patient.

In certain aspects, the probiotic is any of the genera of probiotic bacteria. Examples of probiotics include, but are not limited to, the *Lactobacilli* and *Lactococci* genera, which are used in yougurts and probiotic supplements in the USA, and would serve well as a safe delivery system to treat disease. Strain specificity may be selected based on the organism in which the treatment is occurring (i.e. a mouse specific strain may be selected for mouse *in-vivo* experimentation, while a human specific strain may be selected for human clinical trials and human treatment).

Because amino acid absorption occurs in the small intestine, a probiotic may be selected that is metabolically active in, and preferentially lives in the small intestine. Strains of *Lactobacillus reuteri* exist for mouse and human, and the microbe adheres to mucus of small intestine in both cases. Finally, a high *in vivo* retention rate of foreign DNA in this organism can be achieved by using a specific plasmid section in the final shuttle vector. Integration of the desired gene into the chromosome may also be performed if desired.

The enzyme for phenylalanine (phe) degradation may be selected from the enzyme family of Phenylalanine Ammonia Lysases (PALs). PAL enzymes cleave phenylalanine into trans-cinnamic acid and ammonia, both of which are safely and readily cleared from the mammalian body. Unlike other phe catabolizing enzymes, PAL enzymes do not require co-factors or co-enzymes to fuction. Optimal pH ranges for PAL enzymes are also compatible with proposed physiological pH of the intestine. The multiple potential sources of PAL include plants such as parsley (*Petroselinum crispum*), eubacteria (*Streptomyces maritimus*), cyanobacteria (*Anabaena variabilis, Nostoc punctiforme*) and yeast (*Rhodoturula glutinis*) or other as yet un-discovered sources.

The selected PAL gene may be codon optimized to allow for successful synthesis (translation) of the enzyme in its probiotic host. This is because each organism has a different codon usage frequency for translating mRNAs into proteins/enzymes, and the PAL gene to be inserted must comply with the host codon usage rather than that of its donor. Each organism typically has multiple codon choices available for each amino acid (AA). Therefore, there are multiple potential DNA sequences that may be used in any particular organism that will result in the same AA sequence while still complying with the probiotic host's codon usage.

Modifications in front of the gene selected for the GMP system may be generated to regulate transcription, translation, and location of the translated PAL enzyme. Additionally, a transcriptional terminator may be added to the end of the gene. Restriction sites may be added as needed throughout the gene. In general, known protocols will be used for DNA amplification, electrophoresis and isolation. In some cases, gel electrophoresis may be conducted in an agarose gel loaded in a "mirror" pattern, such that the same samples are loaded in wells equidistant from an imaginary line running down the center of the gel. After running the gel, the gel will be cut in half along the center of the "mirror". One half will be stained with Ethidium bromide and visualized. The visualized bands will be measured for distance from the load point, and that measurement will be used to cut out the band on the unstained (Ethidium bromide free) half of the gel. This reduces the likelihood of spontaneous mutation during handling.

***Transcription:*** A promoter with constitutive expression may be selected and placed in front of the PAL gene in order to drive transcription of the gene. The promoter may be active *in vivo,* or both *in vivo* and *in vitro.* Several examples of good constitutive promoters in *Lactobacilli* and *Lactococci* include, but are not limited to: *ermB, ldhL, lacA,* and *slp*. It is also possible to place a promoterless PAL gene in tandem behind a gene with a functional promoter as long as the gene with a functional promoter does not contain an intervening termination sequence. If the gene with a promoter does contain a termination sequence, the PAL gene must disrupt the original termination sequence while replicating the termination sequence at the end of PAL.

***Translation:*** Following the promoter there may be a ribosome binding site (RBS) with an appropriate length of spacer prior to the start codon. Although there are some general similarities of RBS sequences and spacer length amongst prokaryotes, each species has its own preferences. For *Lactobacillus* and *Lactococci,* a RBS that is rich in adenine (A) and guanine (G) and is 5-6 bases in length appears to be optimal. The spacer following the RBS and ending at the start codon tends to be between 7 and 13 bases long.

***Enzyme location**:* If it becomes desirable to secrete PAL to the cell's exterior, a secretion tag from another enzyme may be added. Examples include, but are not limited to, the secretion tags from the genes Lp_0373, *amyL, nlpl, asp45,* and *mub.*

The final gene construct for the GMP system must be introduced into the probiotic in a stable manner. This may be done by placing a PAL gene construct into an existing high retention plasmid or high retention shuttle plasmid (with or without a selection cassette), synthetically creating a high retention plasmid/shuttle plasmid, homologously recombining the PAL gene into the probiotic's chromosome, phage transduction, or usage of a transposable element. A high retention plasmid is designed to be retained in the bacteria for several generations in the absence of selective pressure. If selection is used in the mouse version of this treatment, an antibiotic resistance gene may be used, such as an ampicillin or erythromycin resistance gene. In some embodiments, erythromycin resistance genes C, B, or GT may be used. An *E. coli* origin of replication to the plasmid may be used, such as the origin in pUC19 (originally from pMB1). In a human version of this treatment, and FDA approved selection cassette would be used, as antibiotic resistance is not acceptable in human probiotics.

After transformation of the probiotic with the gene construct, *in vitro* phe metabolism by the GMP can be assessed by testing for an increase in absorbance at a wavelength of 280nm indicating increase in trans-cinnamic acid production.

The genetically modified probiotic "PHEnominal" organism disclosed herein will have the ability to live in the small intestine, where it will break down phenylalanine (phe) prior to its absorption into the blood stream. By preventing excess phenylalanine from entering the blood stream, phenylalanine toxicity will be prevented.

In certain embodiments, the probiotic organism of the present invention will be delivered to an animal or patient as a purified microbe free of any culture media. Purified probioitic organisms may be administered in a pill, powder or drops. These methods of administration have the advantage that a higher quantity of the organism may be delivered than with a culture medium such as yogurt (i.e. the potential quantity of yogurt to be ingested could be much more than would typically be consumed). Moreover, a purified probiotic organism such as the present invention does not present problems with lactose or dairy intolerant patients, or infants who are not ready to eat solid foods. Finally, administration by pill, powder or drops has the advantage that the probiotic organism can be administered with a range of foods, and patients will not develop a dislike of a food eaten every day, as could be the case with probiotic delivered in yogurt.

The appropriate dosage of the GMP system can be calculated by measuring the number of live organisms which must be consumed by an animal to be recovered live from the feces. This number can be combined with survival tests of the probiotic to arrive at an approximate dosage.

The genetically engineered probiotic can be tested as a treatment using an animal model, for example the PAHenu2 mouse on the C57BL6 background with the appropriate controls. Blood collection can be used to assess serum phe throughout the experiment. Tissue collection at the end of experiment will allow for other health assessments.

Shipping costs of the probiotic organism of the present invention in a purified and freeze-dried form will be more cost-effective than shipping of probiotic in a culture medium, such as yogurt, which may need to be refrigerated, and which may weigh significantly more. Moreover, the administration of purified probiotic in pill, powder, or drop form, as in the present invention, may allow a greater deree of dosage tailoring from patient to patient and possibly day to day for the same patient than probiotic delivered in culture media such as yogurt.

Patients with PKU have typically not eaten dairy products due to their high phe content. The sudden addition of regular and potentially high amounts of dairy could cause side effects in these patients, which would again favor the administration of purified microbe in pill, powder or drop form, as described herein.

Probiotics are comparatively inexpensive to produce. Because the proposed genetically modified probiotic would be classified as a "drug", it may be more likely to be accepted by insurance companies as a treatment they cover (when compared to synthetic foods). Using a phenylalanine degrading probiotic will likely result in higher treatment compliance, as it will allow patients to maintain a more normal diet and life style and not one in which they would have to ingest distasteful foods. As treatment with "PHEmoninal" would require oral administration, it would not require injections as with the PEG-PAL treatment (see above).

### EXAMPLE 1

### Construction of a GMP System - PHEnominal

The species *Lactobacillus reuteri* is used as the probiotic. Strains of this organism are found in the small intestine of most higher animals including humans and mice. Strains that are human and mouse specific have been identified and studied. For the proof of principal experiments *in-vitro* and the mouse *in-vivo* work, we will use *Lactobacillus reuteri* 100-23C. This strain has no indigenous plasmids and is capable of colonizing the small intestine of *Lactobacillus* free mice. When progressing to human clinical trials, a human specific strain of *L. reuteri* or an alternative probiotic as approved by the FDA will be used.

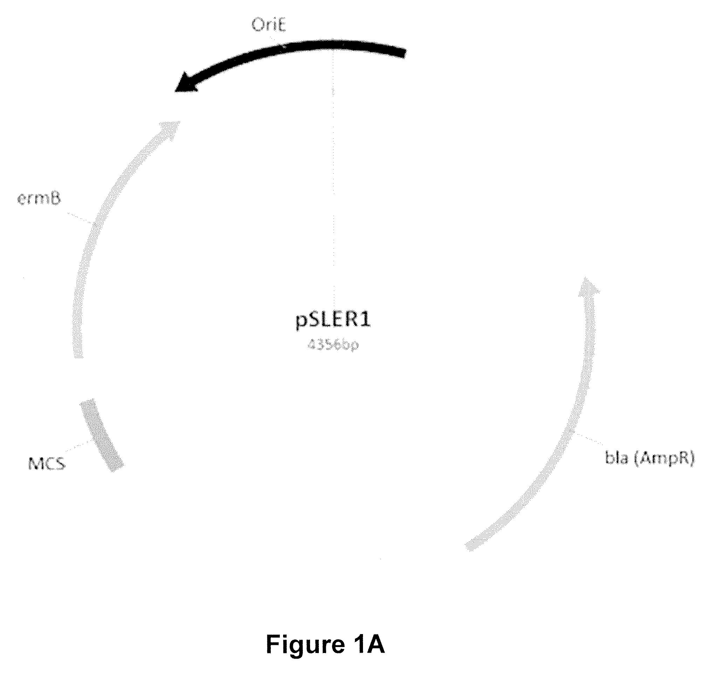

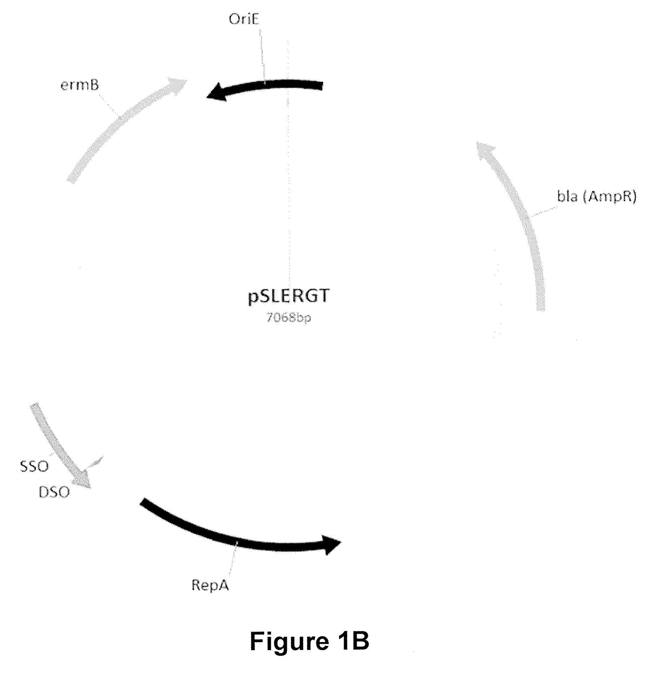

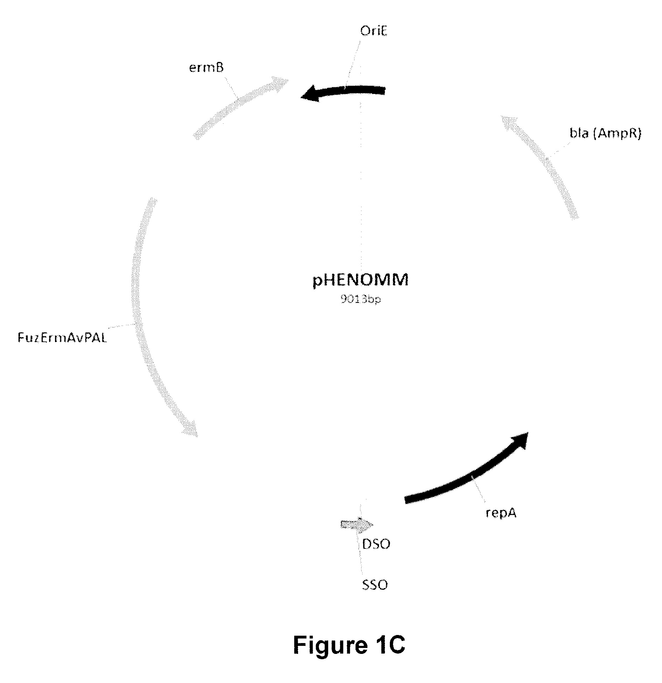

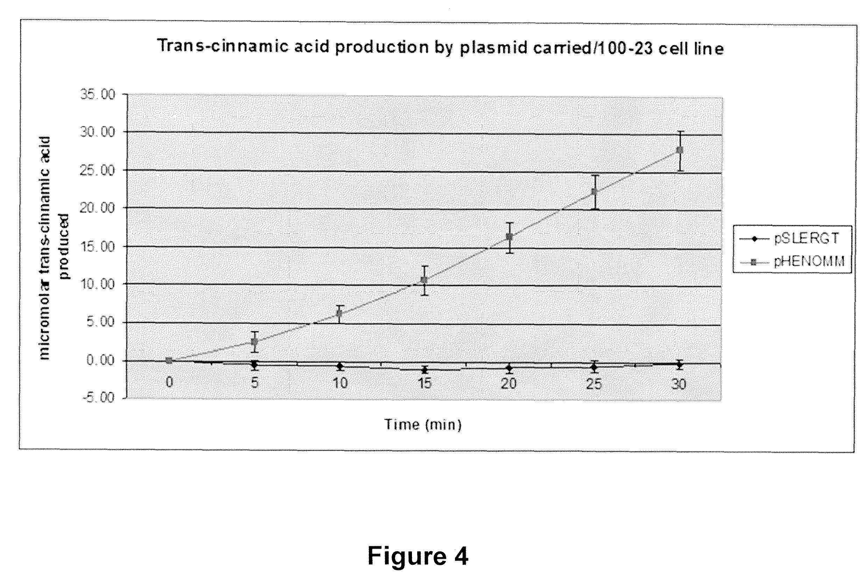

The PAL enzyme to be used is from the cyanobacterium *Anabaena variabilis* ATCC 29413, referred to hereinafter as AvPAL. The sequence for this gene will be codon optimized to match codon usage for the *Lactobacillus reuteri* species as a whole. The AvPAL front sequence (SEQ ID NO: 1) will be placed in front of the codon optimized gene to act as an RBS and spacer sequence. At the end of the gene, the AvPAL end sequence (SEQ ID NO: 2) will be added to act as a transcriptional terminator. This promoterless AvPAL construct (AvPAL plus the above detailed additional sequences) will be synthesized (BioBasic Inc., Ontario, Canada). The complete AvPAL is shown in SEQ ID NO: 3. All sequences not otherwise noted are right facing and in the standard left to right orientation. Plasmids pSLER1, pSLERGT, pHENOMM, and pHENOMM-sec are all left facing and are read right to left. In all plasmids it should be noted that the ermB gene is in the opposite orientation of the plasmid, and this gene is right facing reading left to right (as depicted by plasmid maps in Figures 1A, 1B, and 1C).

A promoter is attached to the AvPAL construct in order to create a functional AvPAL construct. The *ermB* promoter region with 10 additional bases inserted (SEQ ID NO: 4) was selected. This process of fusing the synthesized synthetic *ermB* promoter to AvPAL created the final gene product of FuzErmAvPAL (SEQ ID NO:15) and was performed by BioBasic Inc, Ontario, Canada. Additionally, the secretion tag of Lp_0373 (SEQ ID NO: 5) may be inserted between the ermB promoter and the start codon of the AvPAL gene for secFuzErmAvPAL (SEQ ID NO:6). Once the full construct was created, BioBasic ligated FuzErmAvPAL and secFuzErmAvPAL separately into our synthetically created shuttle vector pSLERGT.

Once FuzErmAvPAL and secFuzErmAvPAL have been synthesised, they must become part of a functional *Lactobacillus reuteri* shuttle plasmid. The shuttle plasmid to be used, pSLERGT (SEQ ID NO: 7), was created specifically for the mouse project by using an antibiotic resistance gene such as erythromycin resistance genes C, B, or GT as the selection cassette, the pGT232 fragment from pNCKH103 (SEQ ID NO: 8) for stability *in-vitro* and *in-vivo* in a *Lactobacillus reuteri* host even in the absence of selection, and *E. coli* origin of replication for shuttle function. The *E. coli* vector pSLER1 (SEQ ID NO: 9) contains the elements *ermB* (erythromycin resistance gene B) and and *E. coli* origin of replication and will be used as the back bone of shuttle vector creation because of these properties. For a human safe plasmid, a selectable gene such as heavy metal resistance or alanine racemase (for complementation) may be used instead of antibiotic resistance. It is also possible in mouse or human safe version to integrate the desired functional AvPAL construct directly into the chromosomal DNA.

To create the desired shuttle vector of pSLERGT, plasmid DNA of pNCKH103 was harvested from 100-23 cells to allow extension PCR of the pGT232 fragment. The reaction used a forward primer for pGT232 amplification (SEQ ID NO: 10) and a reverse primer for pGT232 amplification (SEQ ID NO: 11). The following reaction mixture of New England Biolabs Q5 High Fidelity DNA polymerase was used for a 30µl volume per reaction tube.

| | |
|---|---|
| 10x Q5 polymerase reaction Buffer | 6µl |
| pNCKH103 DNA from L. r. 100-23 | 1.5µl of 15ng/µl total DNA* |
| Forward Primer | 1.5µl |
| Reverse Primer | 1.5µl |
| dNTP 10mM | 2.4µl |
| Water | 18.6µl |
| Q5 DNA polymerase | 0.25µl |

| | |
|---|---|
| * Total DNA, rather than plasmid DNA, is reported due to the dirty quality of DNA extracted from L.r.100-23 cells. | |

**The thermocycler conditions used for amplification were**

| Step | °C | Time (minutes:seconds) |
|---|---|---|
| 1 | 98.0 | 0:30 |
| 2 | 98.0 | 0:10 |
| 3 | 57.0 | 0:12 |
| 4 | 72.0 | 1:30 |
| 5 | go to step 2, x4 | |
| 6 | 98.0 | 0:10 |
| 7 | 65.0 | 0:12 |
| 8 | 72.0 | 1:30 |
| 9 | Go to step 6, x29 | |
| 10 | 72 | 2:00 |
| | 11 4 | ∞ |

The resulting PCR products were then run on a 0.8% agarose gel with 0.5% TBE. Gels run to optimize the reaction conditions were run with ethidium bromide incorporated into the gel. Gels for harvesting DNA for plasmid creation were run in the absence of Ethidium Bromide. For harvesting, the gel was loaded such that the first lane was a DNA ladder, and as many remaining lanes as could be filled contained PCR product. Once the Coomassie blue band reached the end of the gel, electrophoresis was stopped. The gel was cut into 2 sections, so that lanes 1 and 2 were together and the remaining lanes in the second portion. The gel segment with lanes 1 and 2 was stained in a 0.5% TBE ethidium bromide bath in a rocker for 20 minutes to visualize the PCR products with a Fotodyne Incorporated Imager and Foto/Analist PC Image software. The desired pGT232 fragment was excised from this stained portion, and revisualized to confirm appropriate cutting. This stained gel was then used as a guide to cut the appropriate bands from the lanes in the unstained portion of the gel. The bands that were never exposed to ethidium bromide or UV light were used in the UltraClean® GelSpin® DNA Extraction Kit (MoBio Laboratories, Inc., Carlsbad, CA). DNA post gel purification was quantitated with a Nanodrop photospectrometer.

The PCR created pGT232 fragment was cut using NcoI FastDigest (Thermoscientific) for one sticky and one blunt end. The *E. coli* vector/backbone for insertion of the PCR product, pSLER1, was cut using FastDigest enzymes NcoI and SmaI resulting in a sticky blunt vector. Reactants for the reactions as follows
pGT232fragment digest
4µl of pGT232fragment at 17ng/µl
0.5 µl 10x ThermoScientific FastDigest buffer
0.5 µl Thermoscientific FastDigest NcoI enzyme
pSLER1 digest
1.3 µl pSLER1 at 750ng/µl
14.7µl water
2 µl 10x ThermoScientific FastDigest buffer
1 µl Thermoscientific FastDigest NcoI enzyme
1 µl Thermoscientific FastDigest SmaI enzyme

The reactions were incubated in a 37°C water bath for 1.5 hours.

Small DNA fragments created by the digests and the enzymes used were removed from the desired DNA using the solutions protocol of the UltraClean® GelSpin® DNA Extraction Kit (MoBio Laboratories, Inc., Carlsbad, CA) and eluted into molecular water. The resulting DNA concentration was again determined by Nanodrop.

Ligation of the digested, PCR created pGT232 fragment into the sticky blunt pSLER1 vector was done using the T4 DNA ligase from Thermoscientific per the following reaction.
Ligation of pGT232fragment into pSLER1 for creation of pSLERGT
2µl 10x T4 DNA ligase Buffer by ThermoScientific
2µl 50% PEG 4000
1 µl pSLER1 digested at 21ng/µl
5.5µl pGT232 fragment digested at 6ng/µl
8.5µl water

Ligation reaction was at room temperature for 60 min. 5 µl of the ligation reaction was added to 50µl of chemically competent Top10 DH5α *E. coli* from Life Technologies for transformation. Transformation was performed as described by the manufacterer. Transformation reaction was plated onto LB agar containing 50µg/mL of ampicillin.

Colonies from the plates were sub-cultured in TB dry containing 300µg/mL of erythromycin. These liquid cultures were used for the UltraClean 6 minute mini plasmid prep kit (MoBio). Plasmid samples were digested with ThermoScientific FastDigest NcoI and SalI, and the resulting digests were run on a 0.8% agarose 0.5% TBE with ethidium bromide gel to detect clones containing a plasmid with the pGT232 fragment insert.

The clone with successful insertion, the desired pSLERGT, was sent to BioBasic for sequencing. Once the sequencing verified the insert contained no mutations to alter function of the pGT232 fragment (SEQ ID NO:12), pSLERGT was transformed into 100-23C cells to verify functionality of the pGT232 segment in-vitro. pSLERGT successfully transformed 100-23 cells in addition to the *E. coli,* proving it a functional shuttle vector.

The reactants and conditions for electotrotransformation of the 100-23C cells are as described in M. A. McCONNELL et al., "Transfer of Plasmid pAMβ1 Between Members of the Normal Microflora Inhabiting the Murine Digestive Tract and Modification of the Plasmid in a Lactobacillus reuteri Host."

Briefly, a Gene Pulser™' apparatus (Bio-Rad Laboratories, Richmond,CA) will be used for all electroporation experiments described in this study. Recipient cells from overnight cultures at 37°C in MRS broth will be used to inoculate MRS broth to an optical density of 0.06 at 600 nm (OD). Cultures will be incubated at 37°C until an OD 600 of 0.8-1.0 is attained. The cells will be harvested by centrifugation, washed twice in electroporation buffer, and then resuspended in electroporation buffer at 1/20th of the original culture volume. Electroporation buffer consists of 952mM sucrose-3.5mM MgCl₂ at pH 7.2 that has been filter sterilized.

Plasmid DNA, ≥ 1µg, will be added to 0.4ml of cells. This mixture is then placed in chilled sterile electroporation cuvettes (0.2cm inter-electrode gap) and held on ice for 5 min. Following the application of a high-voltage electric pulse, voltage of 12,500 V/cm, 200ohm parallel resistance, 25µFD capacitance, the DNA-cell mixture is added to 10mL of pre-warmed non-selective media for a 3 hour recovery period at 37°C. After recovery, cells are harvested by centrifugation and resuspended in 1mL of media for plating. Approximately 50ul of this solution will be plated onto selective media with 5µg/mL of antibiotic. Diluted aliquots will also be plated on media without antibiotic to ensure cells remained viable throughout the procedure regardless of their plasmid uptake. After incubation of the plates in an anaerobe jar at 37°C for approximately 40h, colonies will be counted/utilized.

Colonies that grow will be positive for the plasmid, and these colonies will be grown in an MRS broth culture in the anaerobe jar with its gas pack components at 37 °C over night. Growth from these MRS overnight cultures will be our stock culture, and as such they will be prepared by using a 1:1 ratio of culture to 10% sterile skim milk and saved by freezing at -80 °C.

The final shuttle vector pSLERGT was sent to BioBasic Inc. as mentioned above for ligation of the final FuzErmAvPAL or secFuzErmAvPAL into pSLERGT to create the therapeutic test vectors pHENOMM (SEQ ID NO:13) and pHENOMM-sec (SEQ ID NO:14) respectively.

The resulting plasmid (pHENOMM or pHENOMH) carried by *L. reuteri* are considered an embodiment of the present invention. Probiotic carrying pHENOMM or pHENOMH will be called PHEnominal. Strain specificity of the probiotic and plasmid used denotes its human or mouse usage. Stock vials of the appropriate strain will be thawed and cultured in MRS broth and anaerobic conditions at 37 °C overnight for *in*-*vitro* and *in-vivo* experimentation. A single vial of stock culture may be used for a long period of study by subculturing the most recent growth of PHEnominal.

### EXAMPLE 2

### Administration and use of the oral PHEnominal to treat PKU

In certain embodiments, the genetically engineered probiotic to treat PKU described herein may be orally administered with food in mice and humans. Survival of the genetically modified probiotic to treat PKU ("PHEnominal") will be greater if it is ingested with food and this will be enhanced further if dairy is ingested concomitant with food. Modes of administration of the oral treatment are varied and may be in pill form, or powder/liquid form. The most appropriate form would be selected for the age and life stage of the patient (e.g. toddlers and infants often resist swallowing pills).

A patient may be provided with access to a phenylalanine monitor to determine levels of phe in the blood. As an essential amino acid, the patient must ensure they are not taking too much of the genetically engineered probiotic. Such phenylalanine monitors for home use are currently in clinical trials, though more expensive versions already exist (approximately $40,000.00 for the currently available machine).

### EXAMPLE 2

At this time, testing of mouse PHEnominal has been performed to demsonstrate functional AvPAL enzyme within the cells. Lysate from 100-23 cells carrying pHENOMM (bearing the FuzErmAvPAL gene, non-secreted) plasmid contain far higher production of trans-cinnamic acid than lysate from cells carrying the empty vector pSLERGT. A representative set of results are seen in Figure 2, and the data retains this trend over multiple experimental replicates. Values depicted indicate the amount of increase in absorbance readings at 280nm, the absorbance point for trans-cinnamic acid/trans-cinnamate, in lysates given phosphate buffered saline with phenylalanine over the amount produced by cells only given phosphate buffered saline alone. Subtracting the values for phosphate buffered saline alone from values measured in the presence of phenylalanine accounts for any increase to the absorbance created by other reactions. These results demonstrate *Lactobacillus reuteri* cells are capable of surviving while producing functional AvPAL enzyme.

### NON-PATENT LITERATURE

Bourget, L, Chang, T. Phenylalanine ammonia-lyase immobilized in semipermeable mircrocapsules for enzyme replacement in phenylketonuria. Federation of European Biochemical Societies, 1985 vol 180 number 1 pp 5-9.
Bourget L, Chang T M S. Biochim Biophys Acta. 1986;883:432-438
Safos S, Chang T M S. Artif Cells Blood Substit Immobil Biotechnol. 1995;23:681-692 Sarkissian C N, Lee K C, Danagher P, Leung R, Fuller M A, Scriver C R. Am J Hum Genet Suppl. 1996;59:1183. (abstr.).
Sarkissian C N, Shao Z, Blain F, Peevers R, Su H, Fuller M A, Scriver C R. Am J Hum Genet Suppl. 1997;61:182. (abstr.).
Liu et al. Study on a Novel Strategy to Treatment of Phenylketonuria. Artif cells, Blood Substit Immobil Biotechnol, 2002, 30(4), 243-257. http://www.pkunsw.org.au/research-blog-full-article
M. A. McCONNELL et al., "Transfer of Plasmid pAMβ1 Between Members of the Normal Microflora Inhabiting the Murine Digestive Tract and Modification of the Plasmid in a Lactobacillus reuteri Host"

## Claims

1. A composition comprising:
a) a probiotic bacterium wherein the probiotic bacterium is *Lactobacillus reuteri;* and
b) a pHENOMM vector having SEQ ID NO: 13 or SEQ ID NO: 14, wherein the pHENOMM vector encodes a gene capable of expressing a functional enzyme for phenylalanine degradation by the probiotic bacterium.

2. The composition of claim 1, wherein the gene capable of expressing a functional enzyme for phenylalanine degradation is cyanobacterium *Anabaena variabilis* ATCC 29413.

3. The composition of claim 1, wherein the gene capable of expressing a functional enzyme for phenylalanine degradation is codon-optimized.

4. The composition of claim 1, wherein the gene capable of expressing a functional enzyme for phenylalanine degradation includes a promoter.

5. The composition of claim 4, wherein the promoter exhibits constitutive expression.

6. The composition of claim 1, wherein the gene capable of expressing a functional enzyme for phenylalanine degradation includes a secretion sequence.

7. The composition of claim 1, wherein the gene capable of expressing functional enzyme for phenylalanine degradation comprises SEQ ID NO: 15, or a functional variant thereof.

8. A composition of claim 1 in for use in a method for treating phenylketonuria.

9. A composition for the use of claim 8 wherein the composition is adapted for oral administration.

## Patentansprüche

1. Zusammensetzung, umfassend:
a) ein probiotisches Bakterium, wobei das probiotische Bakterium *Lactobacillus reuteri* ist; und
b) einen pHENOMM-Vektor, der SEQ ID NO:13 oder SEQ ID NO:14 aufweist, wobei der pHENOMM-Vektor ein Gen codiert, das in der Lage ist, ein funktionelles Enzym für den Phenylalaninabbau durch das probiotische Bakterium zu exprimieren.

2. Zusammensetzung nach Anspruch 1, wobei das Gen, das in der Lage ist, ein funktionelles Enzym für den Phenylalaninabbau zu exprimieren, das Cyanobakterium *Anabaena variabilis* ATCC 29413 ist.

3. Zusammensetzung nach Anspruch 1, wobei das Gen, das in der Lage ist, ein funktionelles Enzym für den Phenylalaninabbau zu exprimieren, codonoptimiert ist.

4. Zusammensetzung nach Anspruch 1, wobei das Gen, das in der Lage ist, ein funktionelles Enzym für den Phenylalaninabbau zu exprimieren, einen Promotor einschließt.

5. Zusammensetzung nach Anspruch 4, wobei der Promotor konstitutive Expression zeigt.

6. Zusammensetzung nach Anspruch 1, wobei das Gen, das in der Lage ist, ein funktionelles Enzym für den Phenylalaninabbau zu exprimieren, eine Sekretionssequenz einschließt.

7. Zusammensetzung nach Anspruch 1, wobei das Gen, das in der Lage ist, ein funktionelles Enzym für den Phenylalaninabbau zu exprimieren, SEQ ID NO:15 oder eine funktionelle Variante davon umfasst.

8. Zusammensetzung nach Anspruch 1 zur Verwendung in einem Verfahren zur Behandlung von Phenylketonurie.

9. Zusammensetzung zur Verwendung nach Anspruch 8, wobei die Zusammensetzung für die orale Verabreichung angepasst ist.

## Revendications

1. Composition comprenant :
a) une bactérie probiotique dans laquelle la bactérie probiotique est *Lactobacillus reuteri ;* et
b) un vecteur pHENOMM ayant SEQ ID NO : 13 ou SEQ ID NO : 14, dans laquelle le vecteur pHENOMM code pour un gène capable d'exprimer une enzyme fonctionnelle pour la dégradation de la phénylalanine par la bactérie probiotique.

2. Composition selon la revendication 1, dans laquelle le gène capable d'exprimer une enzyme fonctionnelle pour la dégradation de la phénylalanine est la cyanobactérie *Anabaena variabilis* ATCC 29413.

3. Composition selon la revendication 1, dans laquelle le gène capable d'exprimer une enzyme fonctionnelle pour la dégradation de la phénylalanine est un gène codon-optimisé.

4. Composition selon la revendication 1, dans laquelle le gène capable d'exprimer une enzyme fonctionnelle pour la dégradation de la phénylalanine comporte un promoteur.

5. Composition selon la revendication 4, dans laquelle le promoteur présente une expression constitutive.

6. Composition selon la revendication 1, dans laquelle le gène capable d'exprimer une enzyme fonctionnelle pour la dégradation de la phénylalanine comporte une séquence de sécrétion.

7. Composition selon la revendication 1, dans laquelle le gène capable d'exprimer une enzyme fonctionnelle pour la dégradation de la phénylalanine comprend SEQ ID NO : 15, ou un variant fonctionnel de celle-ci.

8. Composition selon la revendication 1, pour son utilisation dans un procédé de traitement de la phénylcétonurie.

9. Composition pour son utilisation selon la revendication 8, dans laquelle la composition est appropriée pour une administration orale.
